# EUROPEAN PATENT APPLICATION

(11) **EP 3 653 710 A1**
(43) Date of publication of application: **20.05.2020**
(21) Application number: 18832053.5
(22) Date of filing: 12.07.2018
(51) Int. Cl.: C12N 15/11, C12Q 1/04, C12Q 1/68

(54) **HIGH EXPRESSION mRNA SWITCH**

(30) Priority: 12.07.2017 JP 2017136469
(71) Applicant: Kyoto University, Kyoto-shi, Kyoto 606-8501 (JP)
(72) Inventor: SAITO, Hirohide, Kyoto-shi Kyoto 606-8501 (JP); YI, Kuang, Kyoto-shi Kyoto 606-8501 (JP); PARR, Callum, Kyoto-shi Kyoto 606-8501 (JP)
(74) Representative: HGF Limited
(86) International application number: PCT/JP2018/026362
(87) International publication number: WO 2019/013294

(57) **Abstract**

The present invention provides an mRNA switch which has improved expression level and sensitivity. An mRNA comprising: (i) a nucleic acid sequence specifically recognized by a miRNA or protein; and (ii) a nucleic acid sequence corresponding to a coding region for a marker protein, wherein a nucleotide contained in the mRNA comprises N¹-methyl pseudouridine.

SEQUENCE LISTING

<110>  KYOTO UNIVERSITY

<120>  high expression mRNA switch

<130>  2879-PCT

<150>  JP2017-136469
<151>  2017-07-12

<160>  61

<170>  PatentIn version 3.5

<210>  1
<211>  12
<212>  RNA
<213>  Artificial

<220>
<223>  boxC motif

<400>  1
ggcgugauga gc                                                          12

<210>  2
<211>  34
<212>  RNA
<213>  Artificial

<220>
<223>  kink-loop

<400>  2
agauccgggu gugaacggug aucacccgag aucc                                  34

<210>  3
<211>  45
<212>  RNA
<213>  Artificial

<220>
<223>  kink-loop2

<400>  3
agauccggac guacguguga acggugauca cguacgccga gaucc                      45

<210>  4
<211>  37
<212>  RNA
<213>  Artificial

<220>
<223>  Threonyl-tRNA synthetase binding sequence

<400>  4
ggcguaugug aucuuucgug ugggucacca cugcgcc                               37

<210>  5
<211>  45
<212>  RNA
<213>  Artificial

<220>
<223>  R9-2

<400>  5
cuucgagcgu aggaagaaag ccgggggcug cagauaaugu auagc                    45


<210>  6
<211>  42
<212>  DNA
<213>  Artificial

<220>
<223>  TAP_IVTrev

<400>  6
gccccgcaga aggtctagat cactcgagat gcatatgaga tc                       42


<210>  7
<211>  35
<212>  DNA
<213>  Artificial

<220>
<223>  TAPEGFP_IVTfwd

<400>  7
caccggtcgc caccatggga tccgtgagca agggc                               35


<210>  8
<211>  29
<212>  DNA
<213>  Artificial

<220>
<223>  TAPtagBFP_ IVTfwd

<400>  8
caccggtcgc caccatggga tccagcgag                                      29


<210>  9
<211>  35
<212>  DNA
<213>  Artificial

<220>
<223>  TAPiRFP_IVT fwd

<400>  9
caccggtcgc caccatggcg cgtaaggtcg atctc                               35


<210>  10
<211>  42
<212>  DNA

<213>  Artificial

<220>
<223>  TAPiRFP_IVT rev

<400>  10
gccccgcaga aggtctagat cactcgagat gcatatgaga tc                          42


<210>  11
<211>  36
<212>  DNA
<213>  Artificial

<220>
<223>  hmAG1_IVTfwd

<400>  11
caccggtcgc caccatggtg agcgtgatca agcccg                                 36


<210>  12
<211>  39
<212>  DNA
<213>  Artificial

<220>
<223>  hmAG1_IVTrev

<400>  12
gccccgcaga aggtctagat tcacttggcc tggctgggc                              39


<210>  13
<211>  28
<212>  DNA
<213>  Artificial

<220>
<223>  TAPMS2CP_IVTfwd

<400>  13
caccggtcgc caccatggga tccgcttc                                          28


<210>  14
<211>  30
<212>  DNA
<213>  Artificial

<220>
<223>  Lin28_Tfwd

<400>  14
gccaccatgg gatccggctc cgtgtccaac                                        30


<210>  15
<211>  28
<212>  DNA
<213>  Artificial

<220>

<223>  Lin28_Trev

<400>  15
tatgagatct attctgtgcc tccgggag                                          28


<210>  16
<211>  39
<212>  DNA
<213>  Artificial

<220>
<223>  L7Ae_IVTfwd

<400>  16
caccggtcgc caccatgtac gtgagatttg aggttcctg                              39


<210>  17
<211>  39
<212>  DNA
<213>  Artificial

<220>
<223>  L7Ae Rev primer

<400>  17
ggggggaatt ctcacttctg aaggccttta atcttctcc                               39


<210>  18
<211>  38
<212>  DNA
<213>  Artificial

<220>
<223>  5'ALP orf

<400>  18
caccggtcgc caccatgctg gggccctgca tgctgctg                               38


<210>  19
<211>  45
<212>  DNA
<213>  Artificial

<220>
<223>  3' ALP orf new

<400>  19
gccccgcaga aggtctagac tagggagcag tggccgtctc cagca                       45


<210>  20
<211>  39
<212>  DNA
<213>  Artificial

<220>
<223>  ALPP_rev_2

<400>  20

gccccgcaga aggtctagac tagtcggtgg tgccggcgg                                39

<210>  21
<211>  44
<212>  DNA
<213>  Artificial

<220>
<223>  ALP_ORF_fwd

<400>  21
gcccagccgg ccagatctat catcccagtt gaggaggaga accc                         44

<210>  22
<211>  41
<212>  DNA
<213>  Artificial

<220>
<223>  ALP_ORF_rev

<400>  22
agaaccacca ccagaaccac caccgtcggt ggtgccggcg g                            41

<210>  23
<211>  41
<212>  DNA
<213>  Artificial

<220>
<223>  SignalPeptide_IVT_fwd

<400>  23
caccggtcgc caccatggag acagacacac tcctgctatg g                            41

<210>  24
<211>  18
<212>  DNA
<213>  Artificial

<220>
<223>  SignalPeptide_IVT_rev

<400>  24
agatctggcc ggctgggc                                                      18

<210>  25
<211>  59
<212>  DNA
<213>  Artificial

<220>
<223>  PDGFR_IVT_fwd

<400>  25
ggtggtggtt ctggtggtgg ttctgaacaa aaactcatct cagaagagga tctgaatgc         59

<210>  26
<211>  50
<212>  DNA
<213>  Artificial

<220>
<223>  PDGFR_IVT_rev

<400>  26
gccccgcaga aggtctagac tactaacgtg gcttcttctg ccaaagcatg                50


<210>  27
<211>  77
<212>  DNA
<213>  Artificial

<220>
<223>  hsa-miR-661

<400>  27
cgactcacta taggttccgc gatcgcggat ccacgcgcag gccagagacc caggcaagat      60

caccggtcgc caccatg                                                     77


<210>  28
<211>  77
<212>  DNA
<213>  Artificial

<220>
<223>  hsa-miR-335

<400>  28
cgactcacta taggttccgc gatcgcggat ccacattttt cgttattgct cttgaagatc      60

caccggtcgc caccatg                                                     77


<210>  29
<211>  77
<212>  DNA
<213>  Artificial

<220>
<223>  hsa-miR-210

<400>  29
cgactcacta taggttccgc gatcgcggat cctcagccgc tgtcacacgc acagagatca      60

caccggtcgc caccatg                                                     77


<210>  30
<211>  77
<212>  DNA
<213>  Artificial

<220>
<223>  hsa-miR-21-5p

<400>  30

cgactcacta taggttccgc gatcgcggat cctcaacatc agtctgataa gctaagatca 60

caccggtcgc caccatg 77

<210> 31
<211> 143
<212> DNA
<213> Artificial

<220>
<223> hsa-miR-21-5p

<400> 31
cgactcacta taggttccgc gatcgcggat cctcaacatc agtctgataa gctatcaaca 60

tcagtctgat aagctatcaa catcagtctg ataagctatc aacatcagtc tgataagcta 120

agatcacacc ggtcgccacc atg 143

<210> 32
<211> 77
<212> DNA
<213> Artificial

<220>
<223> hsa-miR-302a

<400> 32
cgactcacta taggttccgc gatcgcggat ccagcaagta catccacgtt taagtagatc 60

caccggtcgc caccatg 77

<210> 33
<211> 147
<212> DNA
<213> Artificial

<220>
<223> hsa-miR-302a

<400> 33
cgactcacta taggttccgc gatcgcggat ccagcaagta catccacgtt taagtagcaa 60

gtacatccac gtttaagtag caagtacatc cacgtttaag tagcaagtac atccacgttt 120

aagtagatca caccggtcgc caccatg 147

<210> 34
<211> 65
<212> RNA
<213> Artificial

<220>
<223> hsa-miR- 17-5p

<400> 34
ggucagaucc gcuaggaucc cuaccugcac uguaagcacu uugagaucua ccggucgcca 60

ccaug 65

<210>  35
<211>  65
<212>  RNA
<213>  Artificial

<220>
<223>  hsa-miR-206

<400>  35
gguuccuuaa ucgcggaucc ccacacacuu ccuuacauuc caagaucaca ccggucgcca      60

ccaug                                                                  65


<210>  36
<211>  76
<212>  RNA
<213>  Artificial

<220>
<223>  hsa-miR-92a

<400>  36
cgacucacua uaggucagau ccgcuaggau ccacaggccg ggacaagugc aauaagaucu      60

accggucgcc accaug                                                      76


<210>  37
<211>  36
<212>  DNA
<213>  Artificial

<220>
<223>  GCT7CMV_G

<400>  37
gctaatacga ctcactatag gtcagatccg ctagcg                                36


<210>  38
<211>  19
<212>  DNA
<213>  Artificial

<220>
<223>  Rev5UTR for MS2

<400>  38
catggtggcg accggtggg                                                   19


<210>  39
<211>  80
<212>  DNA
<213>  Artificial

<220>
<223>  stbC Fwd primer

<400>  39

gaaattaata cgactcacta tagggggctt agggcgggga tttcgcccac aaggaggtgc 60

ccccggacac ccgcagatcg 80

<210> 40
<211> 80
<212> DNA
<213> Artificial

<220>
<223> stbC spacer

<400> 40
acacccgcag atcgagaaga aggcgaatta agagagaaaa gaagagtaag aagaaatata 60

agacaccggt cgccaccatg 80

<210> 41
<211> 80
<212> DNA
<213> Artificial

<220>
<223> KWC0131_T7-kt-EGFP_Fw

<400> 41
gaaattaata cgactcacta taggatccgt gatcggaaac gtgagatcca cctcagatcc 60

gctaggacac ccgcagatcg 80

<210> 42
<211> 80
<212> DNA
<213> Artificial

<220>
<223> Spacer5UTRFwd

<400> 42
acacccgcag atcgagaaga aggcgaatta agagagaaaa gaagagtaag aagaaatata 60

agacaccggt cgccaccatg 80

<210> 43
<211> 39
<212> DNA
<213> Artificial

<220>
<223> GCT7pro_5UTR

<400> 43
gctaatacga ctcactatag gttccttaat cgcggatcc 39

<210> 44
<211> 139
<212> DNA
<213> Artificial

<220>
<223>  3UTR120A

<400>  44
tttttttttt tttttttttt tttttttttt tttttttttt tttttttttt tttttttttt      60

tttttttttt tttttttttt tttttttttt tttttttttt tttttttttt tttttttttt     120

cctactcagg ctttattca                                                  139


<210>  45
<211>  19
<212>  DNA
<213>  Artificial

<220>
<223>  Fwd3UTR

<400>  45
tctagacctt ctgcggggc                                                   19


<210>  46
<211>  92
<212>  DNA
<213>  Artificial

<220>
<223>  IVT_3prime_UTR

<400>  46
tctagacctt ctgcggggct tgccttctgg ccatgccctt cttctctccc ttgcacctgt      60

acctcttggt ctttgaataa agcctgagta gg                                    92


<210>  47
<211>  79
<212>  DNA
<213>  Artificial

<220>
<223>  Rev3UTR2T20

<400>  47
tttttttttt tttttttttt cctactcagg ctttattcaa agaccaagca gtgaattgta      60

atacgactca ctatagggc                                                   79


<210>  48
<211>  31
<212>  DNA
<213>  Artificial

<220>
<223>  TAP_T73GC

<400>  48
cagtgaattg taatacgact cactataggg c                                     31

<210>  49
<211>  87
<212>  DNA
<213>  Artificial

<220>
<223>  IVT_5prime_UTR

<400>  49
cagtgaattg taatacgact cactataggg cgaattaaga gagaaaagaa gagtaagaag      60

aaatataaga caccggtcgc caccatg                                          87


<210>  50
<211>  37
<212>  DNA
<213>  Artificial

<220>
<223>  Rev5UTR

<400>  50
catggtggcg accggtgtct tatatttctt cttactc                               37


<210>  51
<211>  35
<212>  DNA
<213>  Artificial

<220>
<223>  ORF_puroR_fwd

<400>  51
caccggtcgc caccatgacc gagtacaagc ccacg                                 35


<210>  52
<211>  36
<212>  DNA
<213>  Artificial

<220>
<223>  ORF_puroR_rev

<400>  52
gccccgcaga aggtctagat caggcaccgg gcttgc                                36


<210>  53
<211>  106
<212>  DNA
<213>  Artificial

<220>
<223>  T7-U1utr-EGFP_v2_fw

<400>  53
taatacgact cactatagac agcattgtac ccagagtctg tccccagaca ttgcacctgg      60

cgctgtccgc agatcgagaa gaaggcgaat taagagagaa aagaag                    106

<210>  54
<211>  28
<212>  DNA
<213>  Artificial

<220>
<223>  T7+g_Fw

<400>  54
cagtgaattg taatacgact cactatag                                          28


<210>  55
<211>  18
<212>  DNA
<213>  Artificial

<220>
<223>  YK_T7_fwd short

<400>  55
taatacgact cactatag                                                     18


<210>  56
<211>  28
<212>  DNA
<213>  Artificial

<220>
<223>  YK_T7_rev

<400>  56
cgcaaaacgc ctatagtgag tcgtatta                                          28


<210>  57
<211>  37
<212>  DNA
<213>  Artificial

<220>
<223>  KWC00430_U1Afull ORF_Fw

<400>  57
caccggtcgc caccatggcg gcagttcccg agacccg                                37


<210>  58
<211>  47
<212>  DNA
<213>  Artificial

<220>
<223>  KWC00431_U1Afull ORF_Rv

<400>  58
gccccgcaga aggtctagac tacttcttgg caaaggagat cttcatg                     47


<210>  59

<211>  32
<212>  DNA
<213>  Artificial

<220>
<223>  KWC00405_EGFP mRNA1 ORF_Fw

<400>  59
caccggtcgc caccatggtg agcaagggcg ag                                    32


<210>  60
<211>  48
<212>  DNA
<213>  Artificial

<220>
<223>  KWC00427_EGFP ORF_Rv

<400>  60
gccccgcaga aggtctagac tacttgtaca gctcgtccat gccgagag                   48


<210>  61
<211>  72
<212>  DNA
<213>  Artificial

<220>
<223>  KWC0137_Spacer5UTR_ver2

<400>  61
agatcgagaa gaaggcgaat taagagagaa aagaagagta agaagaaata taagacaccg      60

gtcgccacca tg                                                          72

## Description

### [Technical Field]

The present invention relates to high expression mRNA switches that provide high levels of expression in cells.

### [Background Art]

Tissues or organs of multicellular organisms are composed of many types of cells. A human is composed of as many as 60 trillion (6 x 10¹³) cells, and the number of types thereof, even mature cells alone, is at least 400 types. For these cells, techniques for not only analyzing the functions of individual cells, but also distinguishing and identifying a cell type in cell preparation for medical applications have become important.

The present inventors have developed a microRNA (miRNA)-responsive mRNA that expresses a protein in response to endogenous miRNA, and have further developed a method for distinguishing a desired cell type using the expression of the miRNA as an indicator (see, for example, Patent Document 1). Prior to this, the present inventors have also developed a protein-responsive mRNA that, in response to expression of specific proteins, expresses other proteins (see, for example, Patent Document 2).

Patent Document 1 discloses that it is preferred in the introduction of an mRNA switch, such as miRNA-responsive mRNA or protein-responsive mRNA into cells, that the mRNA switch includes a nucleotide having a modified base such as pseudouridine (Ψ) or 5-methyl cytidine (m5C) instead of uridine and cytidine, which are normally contained in native mRNA. Furthermore, it has been reported that mRNA using a nucleotide having a modified base achieved promotion of reporter gene expression (see, for example, Non-Patent Document 1).

### [Citation List]

### [Patent Document]

[Patent Document 1] WO 2015/105172
[Patent Document 2] JP 2009-142259 A

### [Non-Patent Document]

[Non-Patent Document 1]
Journal of Controlled Release 217 (2015) 337-344

### [Summary of Invention]

### [Technical Problem]

Techniques using mRNA switches can be used in distinguishing and identifying specific cells, or fate control of specific cells, thus further development of these techniques is desirable. In particular, since mRNA switches are introduced into cells and function by expressing an encoded protein or suppressing the expression thereof, it is important to increase the expression level thereof. The expression level of the mRNA switch may vary depending on the encoded protein and the cell type into which it is to be introduced. In particular, even when mRNAs encoding a membrane protein or secretory protein were introduced into cells, the expression levels thereof were sometimes low, and the mRNAs sometimes failed to function well as switches.

The present inventors have performed extensive research and have consequently found that mRNA switches, such as miRNA-responsive mRNAs and protein-responsive mRNAs, can achieve increased expression and reduced immune response by preparing the mRNA switches using predetermined modified bases, and thereby completed the present invention.

That is, the present invention provides the matters described below.
[1] An mRNA comprising: (i) a nucleic acid sequence specifically recognized by a miRNA or protein; and (ii) a nucleic acid sequence corresponding to a coding region for a marker protein, wherein a nucleotide contained in the mRNA comprises N¹-methyl pseudouridine.
[2] The mRNA according to [1], wherein the nucleic acid sequence of (i) is a nucleic acid sequence specifically recognized by a miRNA, and the nucleic acid sequences of (i) and (ii) are linked to each other in the 5' to 3' direction.
[3] The mRNA according to [1], wherein the nucleic acid sequence of (ii) is linked to the 3' side of the nucleic acid sequence of (i), and the mRNA further comprises a translation suppression sequence at the 3' side of the nucleic acid sequence of (ii).
[4] The mRNA according to [1], wherein the nucleic acid sequence of (i) is a nucleic acid sequence specifically recognized by a protein, and the nucleic acid sequences of (i) and (ii) are linked to each other in the 5' to 3' direction.
[5] The mRNA according to any one of [1] to [4], wherein the marker protein is a membrane protein or a secretory protein.
[6] The mRNA of any one of [1] to [4], wherein the marker protein is a fluorescent protein, a luminescent protein, a drug-resistant protein, a cell death-inducible protein, or a cell death-inhibitory protein.
[7] A method for distinguishing a cell, comprising the steps of:
   (1) introducing an mRNA into the cell, wherein the mRNA comprises (i) a nucleic acid sequence specifically recognized by a miRNA or protein and (ii) a nucleic acid sequence corresponding to a coding region for a marker protein, and wherein a nucleotide contained in the mRNA comprises N¹-methyl pseudouridine; and
   (2) distinguishing a cell type using a translation amount of the marker protein as an indicator.
[8] The method according to [7], wherein the marker protein is a membrane protein or a secretory protein.

### [Advantageous Effects of Invention]

The present invention provides highly functional mRNAs that express large amounts of encoded proteins when introduced into cells and that avoid immunity problems. The present invention further provides methods for distinguishing a cell using the mRNAs.

### [Brief Description of Drawings]

[Fig. 1(a)] Figs. 1(a) and (b) are graphs showing the results of expression of EGFP mRNAs each consisting of a nucleotide sequence having native or a varied modified base in 293FT cells.
[Fig. 1(b)] Fig. 1(b) is a graph showing the results of expression of EGFP mRNAs each consisting of a nucleotide sequence having native or a varied modified base in 201B7, 201B7D20, A2780, HeLa, SH-5YSY, and NHDF cells.
[Fig. 2] Fig. 2 is a graph showing the result of expression of three types of alkaline phosphatase mRNAs each consisting of a nucleotide sequence having a varied modified base in cells.
[Fig. 3] Fig. 3 is a graph showing the relationship between time and fluorescence intensity when *in vitro* translations of EGFP mRNAs each consisting of a nucleotide sequence having a varied modified base were performed in Rabbit Reticulocyte Lysate.
[Fig. 4] Fig. 4 is a graph showing the time-dependent expressions of EGFP mRNAs in 239FT cells.
[Fig. 5] Fig. 5 is a graph showing the time-dependent expressions of iRFP670 mRNAs in HeLa cells that express EGFP (HeLa EGFP).
[Fig. 6] Fig. 6 shows time-dependent changes of the intracellular amount of EGFP mRNAs each consisting of a nucleotide sequence having a varied modified base.
[Fig. 7] Fig. 7 shows the amounts of active immune response markers 6 hours after the transfection in 293FT to which EGFP mRNAs and PALP mRNAs each consisting of a nucleotide sequence containing a varied modified base were transfected.
[Fig. 8(a)] Fig. 8(a) is a graph showing the dynamic range of protein-responsive EGFP mRNAs composed of nucleotides that have or do not have a modified base.
[Fig. 8(b)] Fig. 8(b) is a drawing showing a schema of the secondary structures of RNA-protein binding motifs and combinations with a trigger protein specifically binding thereto. L7Ae, MS2CP, and Lin28 were used as the trigger protein.
[Fig. 9(a)] Fig. 9(a) shows that the miR-302a switch (miR-302a-responsive Off switch mRNA) having a m1Ψ modified base exhibits a larger dynamic range compared to that having m5C/Ψ modified bases.
[Fig. 9(b)] Fig. 9(b) is a chart showing the result of transfecting 201B7 iPSCs using a 4×miR-302a switch consisting of nucleotides having m5C/Ψ modified bases.
[Fig. 9(c)] Fig. 9(c) is a chart showing the result of transfecting 201B7 iPSCs using a 4×miR-302a switch consisting of nucleotides having a m1Ψ modified base.
[Fig. 10] Fig. 10 shows that multiple types of miR switches each consisting of nucleotides having a m1Ψ modified base improved the dynamic range in HeLa cells.
[Fig. 11(a)] Fig. 11(a) is charts showing the results of transfecting HeLa cells and 293FT cells. The left panel shows the result of transfection with miR-21-5p-responsive mRNA consisting of nucleotides having m5C/Ψ modified bases, and the right panel shows the result of transfection with miR-21-5p-responsive mRNA consisting of nucleotides having a m1Ψ modified base.
[Fig. 11(b)] The left panel of Fig. 11(b) is a histogram of the separation of HeLa cells and 293FT cells using miR-21-5p-responsive mRNA consisting of nucleotides having m5C/Ψ modified bases. The right panel of Fig. 11(b) is a histogram of the separation of HeLa cells and 293FT cells using miR-21-5p-responsive mRNA consisting of nucleotides having a m1Ψ modified base. Fig. 11(b) indicates that the spatial resolution of HeLa cells and 293FT cells is improved when using mRNA consisting of nucleotides having a m1Ψ modified base.
[Fig. 12] Fig. 12 is a photograph of iPSCs stained with alkaline phosphatase (ALP).
[Fig. 13] Fig. 13 is a photograph of partially differentiated iPSCs stained with alkaline phosphatase (ALP).
[Fig. 14] Fig. 14 is a graph showing Fold Change in 293FT cells of miR-21-5p-responsive EGFP switches each consisting of a nucleotide sequence having native or a varied modified base.
[Fig. 15] Fig. 15 shows the result of examining the melting temperature Tₘ of oligonucleotides containing a modified base. Figs. 15(A), 15(B) and 15(C) show typical first and second derivative reports of a native oligonucleotide, Ψ oligonucleotide, and m1Ψ oligonucleotide, respectively.
[Fig. 16] Fig. 16 shows a relative dynamic range of miR-661-responsive EGFP switch, miR-210-responsive EGFP switch or miR-3 3 5-responsive EGFP switch each having native or a Ψ, m1Ψ, or m5C/Ψ modified base(s) when setting the dynamic range of the switch consisting of the native oligonucleotide being 1.
[Fig. 17] Fig. 17 is a graph showing the amount of miR-302a mimics required to suppress 50% of the expression in 293FT cells of 4xmiR-302a-hmAG mRNA switches having native or a Ψ, m1Ψ or m5C/Ψ modified base(s).
[Fig. 18] Fig. 18 is a graph showing Slicer efficiency in 293FT cells of miR-335-EGFP mRNA switches having native or a Ψ or m1Ψ modified base.
[Fig. 19] Fig. 19 is a graph showing Fold Change in 293FT cells of U1A-responsive EGFP mRNA switches having native or a Ψ, m1Ψ, m5C/Ψ, or m5C/m1Ψ modified base(s).

### [Description of Embodiments]

Hereinafter, the present invention is described in detail by way of embodiments. However, the present invention is not limited to the following embodiments.

According to one embodiment, the present invention relates to mRNA. In particular, the present invention relates to an mRNA comprising: (i) a nucleic acid sequence specifically recognized by a miRNA or protein; and (ii) a nucleic acid sequence corresponding to a coding region for a marker protein, wherein a nucleotide contained in the mRNA comprises N¹-methyl pseudouridine.

The mRNA according to the present embodiment is an mRNA that regulates expression of a marker protein when introduced into cells in response to a specific endogenous miRNA or protein. As used herein, such an mRNA that regulates expression of a marker protein in response to an endogenous substance is referred to as a miRNA-responsive mRNA or protein-responsive mRNA, or alternatively, referred to collectively as an mRNA switch. The miRNA-responsive mRNA includes a miRNA-responsive On-switch mRNA that increases expression level of a marker protein in response to miRNA, and a miRNA-responsive Off-switch mRNA that suppresses expression level of a marker protein in response to miRNA. In addition, an mRNA that expresses a marker protein regardless of endogenous substances when introduced into a cell is referred to as a control RNA.

In the present invention, the cell into which the mRNA switch is introduced is not particularly limited, and any cell may be employed. For example, the cell may be a cell collected from a multicellular species or a cell obtained by culturing an isolated cell. In particular, the cell includes a cell collected from a mammal (e.g., human, mouse, monkey, pig, rat) or a cell isolated from a mammal, or a cell obtained by culturing mammalian cell lines. Examples of a somatic cells include keratinized epithelial cells (for example, keratinized epidermal cell), mucosal epithelial cell (for example, an epithelial cell of the tongue surface), exocrine glandular epithelial cell (for example, a mammary cell), hormone-secreting cell (for example, an adrenal medullary cell), metabolic and storage cell (for example, a hepatocyte), luminal epithelial cell that makes up an interface (for example, a type I alveolar cell), luminal epithelial cell of the inner chain (for example, a vascular endothelial cell), ciliated cell with transport ability (for example, a pulmonary epithelial cell), cell for extracellular matrix secretion (for example, a fibroblast), contractile cell (for example, a smooth muscle cell), blood and immune system cell (for example, a T lymphocyte), sensory cell (for example, a sputum cell), autonomic nervous system neurons (for example, a cholinergic neuron), sensory organs and peripheral neuron support cell (for example, an associated cell), central nervous system neuron and glial cell (for example, an astrocyte), pigment cell (for example, a retinal pigment epithelial cell), and progenitor cell thereof (a tissue progenitor cell). There is no particular limitation on the degree of differentiation of the cell or the age of the animal from which the cell is collected. Whether it is an undifferentiated progenitor cell (including somatic stem cell) or a fully differentiated mature cell, it can be used in the same way as the cell in the present invention. Examples of the undifferentiated progenitor cell include tissue stem cell (somatic stem cell) such as neural stem cell, hematopoietic stem cell, mesenchymal stem cell, and dental pulp stem cell.

The cell in the present invention may be a cell that has been subjected to artificial manipulation after collecting precursor somatic cells. For example, the cell in the present invention may be a cell group including iPS cells prepared from somatic cells, or a cell group obtained after differentiating pluripotent stem cells such as ES cells or iPS cells, in which the cell group may include differentiated cells other than the desired cells. The cell according to the present invention is preferably in a viable state. As used herein, the "cell in a viable state" means a cell in a state in which the metabolic capacity of the cell is maintained. In the present invention, the "cell in a viable state" is a cell that can be used in subsequent applications even after miRNA-responsive mRNA is introduced into the cell, while remaining in a viable state without losing its native properties, in particular, maintaining mitogenicity.

### (1) mRNA switch

### (a) miRNA-responsive Off switch mRNA

In the present invention, the "miRNA-responsive Off switch mRNA" means an mRNA including the following nucleic acid sequences of (ia) and (iia):
(ia) a nucleic acid sequence specifically recognized by a miRNA; and
(iia) a nucleic acid sequence corresponding to a coding region for a marker protein.

The (ia) nucleic acid sequence specifically recognized by a miRNA and the (iia) nucleic acid sequence corresponding to a coding region for a marker are operably linked. The miRNA-responsive Off switch mRNA is sometimes referred to as a miRNA Off switch or miRNA switch.

The "miRNA" as used herein is a non-coding RNA of a short strand (20-25 bases) present in a cell and involved in regulation of gene expression through inhibition of translation from mRNA to protein, and degradation of mRNA. The miRNA is transcribed as a single-stranded pri-miRNA that can take a hairpin loop structure containing miRNA and its complementary strand, and a part of the pri-miRNA is cleaved by an enzyme called Drosha in the nucleus to become a pre-miRNA. After the pre-miRNA is transported outside the nucleus, it is further cleaved by Dicer to become active.

The miRNA of (ia) can be appropriately selected, for the purpose of regulating the expression of a marker protein, particularly suppressing or activating the expression of a marker protein in a specific cell, from miRNAs that are specifically expressed or not specifically expressed in the specific cell. The miRNAs that are specifically expressed include miRNAs that are highly expressed in the specific cells compared to other cells at a ratio of 10% or more, 20% or more, 30% or more, 40% or more, 50% or more, 60% or more, 70% or more, 80% or more, 90% or more, or even higher ratio. Such miRNAs can be appropriately selected from miRNAs registered in database information (for example, http://www.mirbase.org/or http://www.microrna.org/) and/or miRNAs described in the literature listed in the database.

It is preferred in the present invention that the nucleic acid sequence specifically recognized by a miRNA is a sequence completely complementary to the miRNA. The nucleic acid sequence specifically recognized by a miRNA is a sequence that may have a mismatch with the completely complementary sequence, as long as it can be recognized by the miRNA. The mismatch from the completely complementary sequence to the miRNA may be any mismatch as long as the nucleic acid sequence can be normally recognized by the miRNA in desired cells. There may be mismatches of about 40 to 50% in the original function of the cells *in vivo*. Such mismatch is not particularly limited, but examples thereof include mismatches of 1 base, 2 bases, 3 bases, 4 bases, 5 bases, 6 bases, 7 bases, 8 bases, 9 bases or 10 bases, and mismatches of 1%, 5%, 10%, 20%, 30% or 40% of the total recognition sequence. The sequence, in particular, may include a number of mismatches, in particular, in portions other than the seed region, that is a reagion at the 5' side of the target sequence, that corresponds to the portion of about 16 bases at the 3' side of the miRNA, as in the miRNA target sequence in the mRNA contained in cells. The portion of the seed region may have no mismatches, or may have mismatches of 1 base, 2 bases, or 3 bases. Such a sequence may have any base length as long as the base length includes the number of bases that allows RISC to specifically bind, but the length of the sequence is preferably greater than or equal to 18 bases and less than 24 bases, more preferably greater than or equal to 20 bases and less than 22 bases. In the present invention, the nucleic acid sequence specifically recognized by miRNA can be appropriately determined and employed by introducing a miRNA-responsive Off switch mRNA having the nucleic acid sequence into the desired cells and other cells, and confirming that expression of the corresponding marker protein is suppressed only in the desired cells.

The "nucleic acid sequence corresponding to a coding region for a marker protein" of (iia) used herein refers to a nucleic acid sequence that can express a marker protein described below which can be translated in a cell and function as a marker. The marker protein is sometimes referred to as a reporter protein.

The protein that can be translated in the cells and function as a marker may be a protein that can be visualized and quantified with fluorescence, luminescence, or coloring, or by assisting fluorescence, luminescence, or coloring. Examples of the fluorescent protein include, but are not limited to, blue fluorescent proteins such as Sirius and EBFP; cyan fluorescent proteins such as mTurquoise, TagCFP, AmCyan, mTFP1, MidoriishiCyan, and CFP; green fluorescent proteins such as TurboGFP, AcGFP, TagGFP, Azami-Green (for example, hmAG1), ZsGreen, EmGFP, EGFP, GFP2, and HyPer; yellow fluorescent proteins such as TagYFP, EYFP, Venus, YFP, PhiYFP, PhiYFP-m, TurboYFP, ZsYellow, and mBanana; orange fluorescent proteins such as KusabiraOrange (for example, hmKO2) and mOrange; red fluorescent proteins such as TurboRFP, DsRed-Express, DsRed2, TagRFP, DsRed-Monomer, AsRed2, and mStrawberry; and near infrared fluorescent proteins such as TurboFP602, mRFP1, JRed, KillerRed, mCherry, HcRed, KeimaRed (for example, hdKeimaRed), mRasberry, and mPlum.

Examples of the luminescent protein include, but are not limited to, aequorin. Examples of the protein that assists fluorescence, luminescence or coloring include, but are not limited to, enzymes that degrade fluorescent, luminescent or coloring precursors, such as luciferases, phosphatases, peroxidases, β-galactosidases, and β-lactamases. When the protein that assists fluorescence, luminescence or coloring is used as a marker gene in the present invention, the distinguish of a desired cell can be performed by bringing the corresponding precursor into contact with the cell, or introducing the corresponding precursor into the cell.

Other examples of the protein that can function as a marker in a cell include proteins that directly affect the function of the cell. Examples thereof include, but are not limited to, cell growth protein, cytotoxic protein, cell signal factor, drug resistance gene, transcription regulatory factor, translation regulatory factor, differentiation regulatory factor, reprograming inducing factor, RNA binding protein factor, chromatin regulatory factor, membrane protein, and secretory protein. For example, the cell growth protein functions as a marker by causing expression of the protein and proliferation only in cells to be identified. The cytotoxic protein functions as a marker by causing cell death of a cell that expresses the protein, and killing the cell containing or not containing a specific miRNA, thereby indicating cell survival and death. The cell signal factor functions as a marker by getting the cells that express the factor to produce specific biological signals and identifying the signals. Examples of the cytotoxic protein include Bax and Bim. The translation regulatory factor functions as a marker, for example, by recognizing and binding to a tertiary structure of a specific RNA, thereby regulating translation from other mRNAs into proteins. Examples of translation regulatory factors include 5R1, 5R2 (Nat Struct Biol. 1998 Jul; 5(7):543-6), B2 (Nat Struct Mol Biol. 2005 Nov; 12(11):952-7), Fox-1 (EMBO J. 2006 Jan 11; 25(1):163-73.), GLD-1 (J Mol Biol. 2005 Feb 11; 346(1):91-104.), Hfq (EMBO J. 2004 Jan 28; 23(2):396-405), HuD (Nat Struct Biol. 2001 Feb; 8(2):141-5.), SRP19 (RNA. 2005 Jul; 11(7):1043-50), L1 (Nat Struct Biol. 2003 Feb; 10(2):104-8.), L11 (Nat Struct Biol. 2000 Oct; 7(10):834-7.), L18 (Biochem J. 2002 Mar 15; 362(Pt 3):553-60), L20 (J Biol Chem. 2003 Sep 19; 278(38):36522-30.), L23 (J Biomol NMR. 2003 Jun; 26(2):131-7), L25 (EMBO J. 1999 Nov 15; 18(22):6508-21.), L30 (Nat Struct Biol. 1999 Dec; 6(12):1081-3.), LicT (EMBO J. 2002 Apr 15; 21(8):1987-97.), MS2 coat (FEBS J. 2006 Apr; 273(7):1463-75.), Nova-2 (Cell. 2000 Feb 4; 100(3):323-32), Nucleocapsid (J Mol Biol. 2000 Aug 11; 301(2):491-511.), Nucleolin (EMBO J. 2000 Dec 15; 19(24):6870-81.), p19 (Cell. 2003 Dec 26; 115(7):799-811), L7Ae (RNA. 2005 Aug; 11(8):1192-200.), PAZ (PiWi Argonaut and Zwille) (Nat Struct Biol. 2003 Dec; 10(12):1026-32.), RnaseIII (Cell. 2006 Jan 27; 124(2):355-66), RR1-38 (Nat Struct Biol. 1998 Jul; 5(7):543-6.), S15 (EMBO J. 2003 Apr 15; 22(8):1898-908.), S4 (J Biol Chem. 1979 Mar 25; 254(6):1775-7.), S8 (J Mol Biol. 2001 Aug 10; 311(2):311-24.), SacY (EMBO J. 1997 Aug 15; 16(16):5019-29.), SmpB (J Biochem (Tokyo). 2005 Dec; 138(6):729-39), snRNP U1A (Nat Struct Biol. 2000 Oct; 7(10):834-7.), SRP54 (RNA. 2005 Jul; 11(7):1043-50), Tat (Nucleic Acids Res. 1996 Oct 15; 24(20):3974-81.), ThrRS (Nat Struct Biol. 2002 May; 9(5):343-7.), TIS11d (Nat Struct Mol Biol. 2004 Mar; 11(3):257-64.), Virp1 (Nucleic Acids Res. 2003 Oct 1; 31(19):5534-43.), VtslP (Nat Struct Mol Biol. 2006 Feb; 13(2):177-8.), and λN (Cell. 1998 Apr 17; 93(2):289-99.). Among them, more preferred translation regulatory factors are MS2 coat and L7Ae.

Examples of the membrane protein include alkaline phosphatase, surface antigen, and membrane transporter (ion transporter). Examples of the secretory protein include hormones and digestive enzymes. Conventional mRNA switches had a problem that, even when the marker genes are designed to express membrane protein and secretory protein, their expression levels are low, making their functioning as switches insufficient. However, the mRNA switch of the present invention ensures a sufficient expression level.

In the present invention, the (ia) nucleic acid sequence specifically recognized by a miRNA and the (iia) nucleic acid sequence corresponding to a coding region for a marker protein in the miRNA are operably linked to each other. The "operably linked" means that at least one target sequence of the miRNA is included in the 5' UTR and/or 3' UTR of an open reading frame (including the start codon) corresponding to a coding region for a marker protein and/or in the open reading frame. It is preferred that the miRNA-responsive Off switch mRNA has a cap structure (7-methylguanosine 5'-phosphate), an open reading frame corresponding to a coding region for a marker protein, and a poly A tail in the 5' to 3' direction from the 5' end, and includes at least one target sequence of the miRNA in the 5' UTR, the 3' UTR, and/or the open reading frame. The miRNA target sequence in the mRNA may be located in any of the 5' UTR, the 3' UTR, and the open reading frame (3' side of the start codon), and may be present in all of these. Thus, the number of the miRNA target sequence may be one, two, three, four, five, six, seven, eight or more.

In the miRNA-responsive Off switch mRNA, it is preferred that the nucleic acid sequences (ia) and (iia) are linked to each other in this order in the 5' to 3' direction. Here, the number and the type of bases between the cap structure and the miRNA target sequence can be set freely as long as they do not constitute a stem structure or a three-dimensional structure. For example, the number of bases between the cap structure and the miRNA target sequence can be set to 0-50 bases, preferably 10-30 bases. The number and type of bases between the miRNA target sequence and the start codon may be freely set as long as they do not constitute a stem structure or a three-dimensional structure. Their locations can be designed so that the number of bases between the miRNA target sequence and the start codon is 0 to 50 bases, preferably 10 to 30 bases.

In the present invention, it is preferred that the miRNA target sequence in the miRNA-responsive mRNA does not contain an AUG that becomes a start codon. For example, if the target sequence of the miRNA is present in the 5' UTR and the target sequence includes an AUG, the miRNA target sequence is preferably designed to be in-frame in relation to the nucleic acid sequence corresponding to a coding region for a marker protein which is linked to the 3' side of the miRNA target sequence. Alternatively, if the target sequence includes an AUG, the target sequence may be used after converting the AUG in the sequence to GUG. Furthermore, in order to minimize the influence of AUG in the target sequence, the location of the target sequence in the 5' UTR may be changed as appropriate. For example, the location thereof may be designed so that the number of bases between the cap structure and the AUG sequence in the target sequence is 0-60 bases, such as 0-15 bases, 10-20 bases, 20-30 bases, 30-40 bases, 40-50 bases, or 50-60 bases.

When designing an mRNA having the nucleic acid sequences of (ia) and (iia) based on the known nucleic acid sequence of the desired miRNA and marker protein, in the case that the native nucleic acids alone are used, an mRNA composed of nucleotides having four types of bases: adenine (A), cytosine (C), guanine (G), and uracil (U) may be typically designed. In contrast, the mRNA of the present invention is prepared to contain N¹-methyl pseudouridine instead of uridine having uracil as a base in the mRNA designed as described above. N¹-methyl pseudouridine is sometimes expressed as "m1Ψ" or 1 NU The structural formula of the lithium salt of N¹-methyl pseudouridine-5'-triphosphate is shown below.

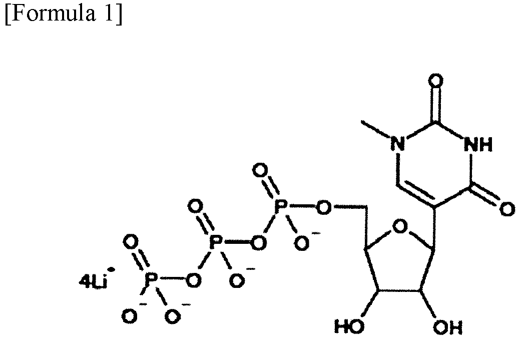

The miRNA-responsive Off switch mRNA may include N¹-methyl pseudouridine instead of all uridines contained in the designed mRNA or may include N¹-methyl pseudouridines in some parts and uridines in other parts. When it includes N¹-methyl pseudouridines in some parts, the N¹-methyl pseudouridines can be present in random positions in any proportion. However, for example, the number of N¹-methyl pseudouridines to the total number of N¹-methyl pseudouridines and uridines is preferably 50% or more, 60% or more, 70% or more, 80% or more, 90% or more, or 95% or more. Most preferably, the miRNA-responsive Off switch mRNA includes N¹-methyl pseudouridines instead of all uridines. The mRNA containing such a modified base can be synthesized by chemical synthesis or genetic engineering techniques, where N¹-methyl pseudouridine-5'-triphosphate is used instead of uridine triphosphate as a nucleic acid to be polymerized. The mRNA switch of the present invention can contain a modified base other than N¹-methyl pseudouridine, such as pseudouridine (Ψ), 5-methylcytidine (m5C), N6-methyladenosine (m6A), or any combination thereof. However, it is preferred that the mRNA switch of the present invention includes no nucleotides having a modified base other than N¹-methyl pseudouridine.

### (b) miRNA-responsive On switch mRNA

In the present invention, the "miRNA-responsive On switch mRNA" means an mRNA including the following nucleic acid sequences (ib), (iib) and (iiib):
(ib) a nucleic acid sequence specifically recognized by a miRNA linked to the 3' side of the polyA sequence,
(iib) a nucleic acid sequence corresponding to a coding region for a marker protein, and
(iiib) a translation suppression sequence linked to the 3' side of the nucleic acid sequence specifically recognized by the miRNA.

The miRNA-responsive On switch mRNA is sometimes referred to as miRNA On switch or miRNA switch.

In the miRNA-responsive On switch mRNA, the nucleic acid sequence specifically recognized by a miRNA in the (ib) and the (iib) nucleic acid sequence corresponding to a coding region for a marker protein can be the same sequences as those defined in the (a) miRNA-responsive Off switch mRNA.

It is preferred that the miRNA-responsive On switch mRNA has a cap structure (7 methylguanosine 5'-phosphate), an open reading frame encoding a desired protein gene, and a PolyA sequence in the 5' to 3' direction from the 5' end, and includes a miRNA target sequence linked to the 3' side of the PolyA sequence and a translation suppression sequence linked to the 3' side of the miRNA target sequence.

The structure of the 5' UTR is not particularly limited in number of bases or base sequence as long as it includes a cap structure at the 5' end and does not have a miRNA target sequence. As an example, the structure of the 5' UTR can be composed of 20 bases or more, for example, 40 to 150 bases, preferably about 40 to 100 bases, and can have a sequence that is difficult to form an RNA structure such as a Stem-loop structure and does not contain a start codon. However, the structure of the 5' UTR is not limited to particular sequences. The PolyA sequence has no specific upper limit in length, and can be, for example, a sequence consisting of 50 to 300 A bases, preferably 100 to 150 A bases.

The number of miRNA target sequences linked to the 3' side of the PolyA sequence is preferably one. This is because if the sequence remains at the 3' side of the PolyA after cleavage of the target sequence by miRNA, it will be difficult to be recognized as PolyA, and it may fail to start the gene expression (fail to stop the expression suppression). However, a plurality of miRNA target sequences may be linked to the 3' side of the PolyA sequence if the expression suppression can be released after the target sequences are cleaved by miRNA. The notation "miRNA target sequence linked to the 3' side of the PolyA sequence" means that the miRNA target sequence may be linked directly to the PolyA sequence, or that a sequence that does not affect the function, for example, a sequence of about 1 to 5 bases may be included between the two sequences.

A translation suppression sequence is linked to the 3' side of the miRNA target sequence. In the (iiib), the notation "translation suppression sequence linked to the 3' side of the miRNA target sequence" means that the translation suppression sequence may be linked directly to the miRNA target sequence, or that another sequence may be present between the two sequences. For example, an adapter sequence of 20 to 100 bases may be present between the miRNA target sequence and the translation suppression sequence. When the miRNA-responsive On switch mRNA includes two or more miRNA target sequences, the notation "3' side of the miRNA target sequence" refers to the 3'-end side of the miRNA target sequence located on the most 3' side.

The translation suppression sequence may be a sequence capable of preventing the action of the PolyA sequence involved in the translation. Preferably, the translation suppression sequence includes a base sequence selected from (α) a base sequence of 20 bases or more which specifically recognizes a PolyA sequence, (β) a sequence which specifically recognizes 5' UTR, and (γ) a sequence of 100 bases or more.

Examples of the (α) base sequence of 20 bases or more which specifically recognizes a PolyA sequence include, but are not limited to, a PolyU sequence that is fully complementary to the PolyA sequence, such as, a PolyU sequence composed of 20 or more, 40 or more, 60 or more, or 80 or more uridines. The base sequence of (α) may contain a mismatch as long as it can specifically recognize the PolyA sequence. Furthermore, the base sequence of (α) may further contain any sequence on its 3' side and/or 5' side as long as it contains a base sequence of 20 bases or more that specifically recognizes the PolyA sequence. The base sequence of (α) can be a base sequence of 5 bases or more, 10 bases or more, or 15 bases or more, that is, not requiring 20 bases or more, as long as the sequence can specifically recognize the PolyA sequence and suppress the translation.

The (β) sequence which specifically recognizes 5' UTR is preferably a sequence that is completely complementary to the 5' UTR. However, the sequence of (β) may have a mismatch with the completely complementary sequence as long as it specifically recognizes the 5' UTR. Furthermore, the sequence of (β) may further contain any sequence on its 3' side and/or 5' side as long as it contains a sequence which specifically recognizes the 5' UTR.

The (γ) sequence of 100 bases or more may be any sequence that is long enough to suppress the translation, regardless of the type and sequence of the bases. The sequence of (γ) may be a sequence of 100 bases or more, preferably 300 bases or more, 500 bases or more, 1000 bases or more, or 1500 bases or more.

The miRNA-responsive On switch mRNA having such structural features can hide the polyA sequence from the endogenous translation system and inhibit the translation. While mRNAs in cells are assumed that they are recognized and activated to translate through the CAP structure present at the 5' end and the polyA sequence present at the 3' end, the artificial miRNA-responsive On switch mRNAs designed as described above can be said to be temporarily inactivated. When the artificial miRNA-responsive On switch mRNA is put under the presence of miRNA that specifically binds to the target sequence, the translation suppression by the translation suppression sequence is released, and the translation to the marker protein is carried out.

When designing a miRNA-responsive On switch mRNA containing the nucleic acid sequences (ib), (iib) and (iiib) as described above, in the case that the native nucleic acids alone are used, an mRNA composed of nucleic acids having four types of bases: adenine (A), cytosine (C), guanine (G), and uracil (U) may be designed. In contrast, the mRNA of the present invention is prepared to contain N¹-methyl pseudouridine instead of uridine having uracil as a base in the mRNA designed as described above. The proportion of N¹-methyl pseudouridine and the content of nucleotides having other modified bases may be the same as that described above in the (a) miRNA-responsive Off switch mRNA.

### (c) Protein-responsive Off switch mRNA

In the present invention, the "protein-responsive Off switch mRNA" means an mRNA including the following nucleic acid sequences of (ic) and (iic):
(ic) a nucleic acid sequence specifically recognized by a trigger protein, and
(iic) a nucleic acid sequence corresponding to a coding region for a marker protein.

The (ic) nucleic acid sequence specifically recognized by a trigger protein and the (iic) nucleic acid sequence corresponding to a coding region for a marker protein are operably linked. The protein-responsive Off switch mRNA is sometimes referred to as protein-responsive mRNA or protein-responsive switch.

In the present embodiment, the (ic) nucleic acid sequence specifically recognized by a trigger protein is an RNA containing a sequence that forms an RNA-protein binding motif. The RNA containing a sequence that forms an RNA-protein binding motif is an RNA-potion contained in an RNA-protein binding motif in a native or known RNA-protein complex or an RNA-portion contained in an artificial RNA-protein binding motif obtained by *in vitro* selection method. Then, the trigger protein includes a protein portion contained in the RNA-protein binding motif in a native or known RNA-protein complex.

The sequence forming a native RNA-protein binding motif is usually composed of about 5 to 30 bases, and is known to form a specific bond with a protein having a specific amino acid sequence by non-covalently bonding, that is, hydrogen bonding. Sequences forming such a native RNA-protein binding motif can be obtained from Tables 1 and 2 below, as well as the database available at the website: http://gibk26.bse.kyutech.ac.jp/jouhou/image/dna-protein/RNA/RNA.html by appropriately selecting a motif that produces the desired structural change. The RNA-protein binding motif preferably used in the present embodiment is a motif that has already undergone an x-ray crystallographic structural analysis or NMR structural analysis or a motif of which the three-dimensional structure can be estimated from the three-dimensional structure of a homologous protein that has already undergone a structural analysis. Furthermore, the motif is preferably a motif in which the protein specifically recognizes the secondary structure and base sequence of RNA.

**[Table 1]**

| Name of RNA | Name of protein | Kd | Publication |
|---|---|---|---|
| 5S RNA (*Xenopus laevis* oocyte) | 5R1 | 0.64 ± 0.10 nM | Nat Struct Biol. 1998 Jul;5(7):543-6 |
| 5S RNA (*Xenopus laevis* oocyte) | 5R2 | 0.35 ± 0.03 nM | Nat Struct Biol. 1998 Jul;5(7):543-6 |
| dsRNA | B2 | 1.4 ± 0.13 nM | Nat Struct Mol Biol. 2005 Nov;12(11):952-7 |
| RNA splicing motif with UGCAUGU element | Fox-1 | 0.49 nM at 150 mM salt | EMBO J. 2006 Jan 11;25(1):163-73. |
| TGE | GLD-1 | 9.2 ± 2 nM | J Mol Biol. 2005 Feb 11;346(1):91-104. |
| sodB mRNA | Hfq | 1.8 nM | EMBO J. 2004 Jan 28;23(2):396-405. |
| RyhB (siRNA) | Hfq | 1500 nM | Ann Rev Microbiol. 2004;58:303-28 |
| mRNA | HuD | 0.7 ± 0.02 nM | Nat Struct Biol. 2001 Feb;8(2):141-5 |
| S domain of 7S RNA | human SRP19 | | RNA. 2005 Jul;11(7):1043-50. Epub 2005 May 31 |
| Large subunit of SRP RNA | human SRP19 | 2 nM | Nat Struct Biol. 2001 Jun;8(6):515-20 |
| 23S rRNA | L1 | | Nat Struct Biol. 2003 Feb;10(2):104-8 |
| 23S rRNA | L11 | | Nat Struct Biol. 2000 Oct;7(10):834-7 |
| 5S rRNA | L18 | | Biochem J. 2002 May 1;363(Pt 3):553-61 |
| 23S rRNA | L20 | 13 ± 2 nM | J Biol Chem. 2003 Sep 19;278(38):36522-30. |
| Own mRNA site1 | L20 | 88 ± 23 nM | J Biol Chem. 2003 Sep 19;278(38):36522-30. |
| Own mRNA site2 | L20 | 63 ± 23 nM | Mol Microbiol. 2005 Jun;56(6):1441-56 |
| 23S rRNA | L23 | | J Biomol NMR. 2003 Jun;26(2):131-7 |
| 5S rRNA | L25 | | EMBO J. 1999 Nov 15;18(22):6508-21 |
| Own mRNA | L30 | | Nat Struct Biol. 1999 Dec;6(12):1081-3. |
| mRNA | LicT | | EMBO J. 2002 Apr 15;21(8):1987-97 |
| Own mRNA | MS2 coat | 39 ± 5 nM | FEBS J. 2006 Apr;273(7):1463-75 |
| Stem-loop RNA motif | Nova-2 | | Cell. 2000 Feb 4;100(3):323-32 |
| SL2 | Nucleocapsid | 110 ± 50 nM | J Mol Biol. 2000 Aug 11;301(2):491-511 |
| Pre-rRNA | Nucleolin | | EMBO J. 2000 Dec 15;19(24):6870-81 |
| | p19 | 0.17 ± 0.02 nM | Cell. 2003 Dec 26;115(7):799-811 |
| Box C/D | L7Ae | 0.9 ± 0.2 nM | RNA. 2005 Aug;11(8):1192-200. |
| siRNA with the characteristic two-base 3' overhangs | PAZ(PiWi Argonaut and Zwille) | | Nat Struct Biol. 2003 Dec;10(12):1026-32. |
| dsRNA | Rnase III | | Cell. 2006 Jan 27;124(2):355-66 |
| HIV-1 RRE (IIB) | RR1-38 | 3-8 nM | Nat Struct Biol. 1998 Jul;5(7):543-6 |
| Own mRNA | S15 | 5 nM | EMBO J. 2003 Apr 15;22(8):1898-908 |
| 16S rRNA | S15 | 6 nM | Nat Struct Biol. 2000 Apr;7(4):273-277. |
| Own mRNA | S15 | 43 nM | EMBO J. 2003 Apr 15;22(8):1898-908 |
| 16S rRNA | S4 | 6.5 µM in 4°C, 1.7 nM in 42°C | J Biol Chem. 1979 Mar 25;254(6):1775-7 |
| 16S rRNA | S4 | 18 µM | J Biol Chem. 1979 Mar 25;254(6):1775-7 |
| 16S rRNA | S8 | 26 ± 7 nM | J Mol Biol. 2001 Aug 10;311(2):311-24 |
| mRNA | S8 | 200 nM | RNA. 2004 Jun;10(6):954-64 |
| mRNA | SacY | 1400 nM | EMBO J. 1997 Aug 15;16(16):5019-29 |
| SnRNA | Sm | | Cold Spring Harb Symp Quant Biol. 2006;71:313-20. |
| tmRNA | SmpB | 21 ± 7 nM | J Biochem (Tokyo). 2005 Dec;138(6):729-39 |
| TD3 of tmRNA | SmpB | 650 nM | J Biochem (Tokyo). 2005 Dec;138(6):729-39 |
| U1 snRNA | snRNP U1A | 0.032 ± 0.007 nM (salt dependence) | Nat Struct Biol. 2000 Oct;7(10):834-7 |
| S domain of 7S RNA | SRP54 | 500 nM | RNA. 2005 Jul;11(7):1043-50. |
| TAR | Tat | 200-800 nM | Nucleic Acids Res. 1996 Oct 15;24(20):3974-81 |
| BIV TAR | Tat | 1.3 nM or 8 nM or 60 nM (Changed depending on difference in Mg) | Mol Cell. 2000 Nov;6(5):1067-76 |
| tRNA^{Thr} | ThrRS | 500 nM | Nat Struct Biol. 2002 May;9(5):343-7 |
| *thrS* mRNA operator | ThrRS | 10 nM | Trends Genet. 2003 Mar;19(3):155-61 |
| Single stranded mRNA | TIS11d | | Nat Struct Mol Biol. 2004 Mar;11(3):257-64. |
| PSTVd | Virp1 | 500 nM | Nucleic Acids Res. 2003 Oct 1;31(19):5534-43 |
| RNA hairpin; Smaug recognition element (SRE) | Vts1p | 30 nM | Nat Struct Mol Biol. 2006 Feb;13(2):177-8. |
| λ BoxB | λ N | 90 nM | Cell. 1998 Apr 17;93(2):289-99 |

The RNA containing a sequence that forms an artificial RNA-protein binding motif is an RNA portion in an RNA-protein binding motif in an artificially designed RNA-protein complex. The base sequence of such an RNA is usually composed of about 10 to 80 bases, and is designed to form a specific bond with a specific amino acid sequence of a specific protein by non-covalently bonding, that is, hydrogen bonding. Examples of the RNA containing a sequence that forms such an artificial RNA-protein binding motif include an RNA aptamer that specifically binds to a specific protein. The RNA aptamer that specifically binds to a desired protein to be targeted can be obtained, for example, by evolutionary engineering methods known as *in vitro* selection or SELEX methods. The trigger protein at this time is a protein to which the RNA aptamer binds. For example, the RNA sequences listed in Table 3 below are known. These RNA sequences can also be used as a sequence forming the RNA-protein binding motif of the present invention.

**[Table 3]**

| Name of RNA | Name of protein | Kd | Publication |
|---|---|---|---|
| Rev aptamer 5 | Rev | 190 nM | RNA. 2005 Dec;11(12):1848-57 |
| Aptamer | p50 | 5.4 ± 2.2 nM | Proc Natl Acad Sci U S A. 2003 Aug 5;100(16):9268-73. |
| BMV Gag aptamer | BMV Gag | 20 nM | RNA. 2005 Dec;11(12):1848-57 |
| BMV Gag aptamer | CCMV Gag | 260 nM | RNA. 2005 Dec;11(12):1848-57 |
| CCMV Gag aptamer | CCMV Gag | 280 nM | RNA. 2005 Dec;11(12):1848-57 |
| CCMV Gag aptamer | BMV Gag | 480 nM | RNA. 2005 Dec; 11(12):1848-57 |

In the present embodiment, the sequence forming the RNA-protein binding motif preferably has a dissociation constant Kd with the corresponding trigger protein of about 0.1 nM to about 1 µM.

In addition to the sequences forming the RNA-protein binding motif themselves, variants of these sequences are also encompassed by the sequence according to the present invention. The term "variant" as used herein refers to a variant of which the dissociation constant Kd with a protein that specifically binds to a sequence forming the RNA-protein binding motif is 10%, 20%, 30%, 40% or 50% or more higher, or is 10%, 20%, 30%, 40% or 50% or more lower than that of those sequences. Such variants can be appropriately selected and employed as long as they can form an RNA-protein complex. The base sequence of such variants may be a base sequence capable of hybridizing under stringent conditions with nucleic acids having a sequence (complementary strand) complementary to the sequence (positive strand) forming the RNA-protein binding motif. The stringent conditions here can be determined based on the melting temperature (Tₘ) of the nucleic acid to be bound, as taught by Bergerand Kimmel (1987, Guide to Molecular Cloning Techniques Methods in Enzymology, Vol. 152, Academic Press, San Diego, CA). For example, the washing condition after hybridization can be typically a condition of about "1 × SSC, 0.1% SDS, 37°C". It is preferred that the complementary strand maintains the hybridized state with the positive strand of interest even when washed under such conditions. Examples of the stricter hybridization conditions include, but are not limited to, a condition in which a positive strand and a complementary strand maintain a hybridization state even after being washed in washing conditions of about "0.5 × SSC, 0.1%SDS, 42°C", further strictly, "0.1 × SSC, 0.1% SDS, 65°C". Specifically, the sequence of the variant consists of a base sequence having at least 90%, preferably at least 95%, 96%, 97%, 98% or 99% sequence identity with the RNA sequence contained in the RNA-protein binding motif described above. Such variants can retain a certain binding with a protein that specifically binds to a sequence that forms an RNA-protein binding motif, thereby contribute to the formation of RNA-protein complex.

Specific examples of the sequence that forms an RNA-protein binding motif according to the present embodiment include box C motif (5'-GGCGUGAUGAGC-3') (SEQ ID NO: 1), kink-loop (SEQ ID NO: 2) and kink-loop 2 (SEQ ID NO: 3) as shown in Table 4 below, which are the sequence to which L7Ae (Moore T et al., Structure Vol. 12, pp. 807-818 (2004)) binds.

**[Table 4]**

| RNA name | Sequence (5' -> 3') | Sequence ID No. |
|---|---|---|
| boxC motif | GGCGUGAUGA GC | 1 |
| kink-loop | AGAUCCGGGU GUGAACGGUG AUCACCCGAG AUCC | 2 |
| kink-loop2 | AGAUCCGGAC GUACGUGUGA ACGGUGAUCA CGUACGCCGA GAUCC | 3 |

Other specific examples include MS2 stem loop motif to which MS2 coat protein specifically binds (22: Keryer-Bibens C, Barreau C, Osborne HB (2008) Tethering of proteins to RNAs by bacteriophage proteins. Biol Cell 100: 125-138), and Fr15 to which *Bacillus* ribosomal protein S15 binds (24:Batey RT, Williamson JR (1996) Interaction of the Bacillus stearothermophilus ribosomal protein S15 with 16SrRNA: I. Defining the minimal RNA site. J Mol Biol 261: 536-549).

Further specific examples include a sequence: 5'-GGCGUAUGUGAUCUUUCGUGUGGGUCACCACUGCGCC-3' (SEQ ID NO: 4) to which threonyl-tRNA synthetase bind, and variants thereof. The threonyl-tRNA synthetase is an enzyme that performs aminoacylation and is known to have feedback inhibition by binding to its own mRNA and thereby inhibiting translation (Cell (Cambridge, Mass.) V97, pp. 371-381 (1999)). Still more specific examples include R9-2: 5'-GGGUGCUUCGAGCGUAGGAAGAAAGCCGGGGGCUGCAGAUAAUGUAUAGC-3' (SEQ ID NO: 5), which is a base sequence forming an RNA-protein binding motif derived from Bcl-2 family CED-9 that is a cancer cell-specific endogenous protein, and variants thereof; and a base sequence derived from an aptamer of an RNA sequence that binds to NF-kappa B, and variants thereof.

The (iic) nucleic acid sequence corresponding to a coding region for a marker protein is as described in the (a) miRNA-responsive Off switch mRNA, and can have the same structure as that of (iia) in the miRNA-responsive Off switch mRNA.

Preferably, in the protein-responsive mRNA, the nucleic acid sequences of (ic) and (iic) are linked to each other in this order in the 5' to 3' direction. Here, the number and the type of bases between the cap structure and the nucleic acid sequence specifically recognized by the trigger protein can be set freely as long as they do not constitute a stem structure or a three-dimensional structure. For example, the number of bases between the cap structure and the nucleic acid sequence specifically recognized by the trigger protein can be set to 0-50 bases, preferably 10-30 bases. The number and type of bases between the nucleic acid sequence specifically recognized by the trigger protein and the start codon may be freely set as long as they do not constitute a stem structure or a three-dimensional structure. The number of bases between the nucleic acid sequence specifically recognized by the trigger protein and the start codon may be designed in an arrangement so that it is 0 to 50 bases, preferably 10 to 30 bases.

In the present invention, it is preferred that the nucleic acid sequence specifically recognized by the trigger protein in the protein-responsive mRNA does not contain an AUG that becomes a start codon. For example, when the nucleic acid sequence specifically recognized by the trigger protein is present in the 5' UTR and the nucleic acid sequence includes an AUG, the nucleic acid sequence is preferably designed to be in-frame in relation to the nucleic acid sequence corresponding to a coding region for a marker protein which is linked to the 3' side of the nucleic acid sequence. Alternatively, when the nucleic acid sequence specifically recognized by the trigger protein includes an AUG, the nucleic acid sequence specifically recognized by the trigger protein may be used after converting the AUG in the nucleic acid sequence to GUG. Furthermore, in order to minimize the influence of an AUG in the nucleic acid sequence specifically recognized by the trigger protein, the location of the nucleic acid sequence specifically recognized by the trigger protein in the 5' UTR may be changed as appropriate. For example, the location may be designed so that the number of bases between the cap structure and the AUG sequence in the nucleic acid sequence specifically recognized by the trigger protein is 0-60 bases, such as 0-15 bases, 10-20 bases, 20-30 bases, 30-40 bases, 40-50 bases, or 50-60 bases.

When designing a protein-responsive Off switch mRNA containing the nucleic acid sequences of (ic) and (iic) as described above, in the case that the native nucleic acids alone are used, an mRNA composed of nucleic acids having four types of bases: adenine (A), cytosine (C), guanine (G), uracil (U) may be designed. In contrast, the mRNA of the present invention is prepared to contain N¹-methyl pseudouridine instead of uridine having uracil as a base in the mRNA designed as described above. The proportion of N¹-methyl pseudouridine and the content of nucleotides having other modified bases may be the same as that described above in the (a) miRNA-responsive Off switch mRNA.

When the trigger protein in the protein-responsive Off switch mRNA is not an endogenous protein, an mRNA encoding the trigger protein for controlling the protein-responsive Off switch mRNA may be used concurrently. The mRNA encoding the trigger protein may be an mRNA capable of regulating expression of the trigger protein, or may be an mRNA that does not regulate the expression of the trigger protein. The former mRNA may be an mRNA containing, for example, a nucleic acid sequence specifically recognized by a miRNA and a nucleic acid sequence corresponding to a coding region for the trigger protein where these nucleic acid sequences are operably linked to each other. In this case, the 5' UTR can be designed in the same manner as the 5' UTR of the (a) miRNA-responsive Off switch mRNA. The latter mRNA may be an mRNA that does not contain a nucleic acid sequence specifically recognized by a miRNA but contains a nucleic acid sequence corresponding to a coding region for the trigger protein. In any case, a protein that can specifically recognize the nucleic acid sequence of (ic) in the protein-responsive Off switch mRNA can be designed as a trigger protein. In this case as well, when the native nucleic acids alone are used, an mRNA composed of nucleic acids having four types of bases: adenine (A), cytosine (C), guanine (G), uracil (U) may be designed, but it is preferred that the mRNA is prepared to contain N¹-methyl pseudouridine instead of uridine having uracil as a base. The proportion of N¹-methyl pseudouridine and the content of nucleotides having other modified bases may be the same as those described in the (a) miRNA-responsive Off switch mRNA.

### (2) Cell distinguishing method

According to one embodiment, the present invention provides a method for distinguishing a cell, comprising the steps of:
(1) introducing an mRNA into the cell, wherein the mRNA comprises (i) a nucleic acid sequence specifically recognized by a miRNA or protein and (ii) a nucleic acid sequence corresponding to a coding region for a marker protein, and wherein a nucleotide contained in the mRNA comprises N¹-methyl pseudouridine; and
(2) distinguishing a cell type using a translation amount of the marker protein as an indicator.

In the method according to the present embodiment, "distinguishing" a cell refers to presenting, for a desired specific one or more cell types, detectable signal information different from that of other cell types in a cell group including two or more types of cells. In particular, it refers to presenting visually recognizable information. The visually recognizable information is not limited to information in which a cell produces a signal that can be directly observed, but also includes information in which a signal produced by a cell is converted into visually recognizable information with numerical values, charts, images, or the like, and which can be visually recognized by one skilled in the art. In the present specification, the term "distinguishing" can include, after the distinguishing, recognizing a desired cell type, discriminating a desired cell type, identifying a desired cell type, classifying a desired cell type, isolating a desired cell type, removing unwanted cell types, determining the life or death of a desired cell type, detecting or quantifying specific biological signals of a desired cell type, and fractionating a desired cell type based on specific physical or chemical signals.

In the method according to the present invention, the mRNA switch to be introduced into cells may typically be (a) miRNA-responsive Off switch mRNA, (b) miRNA-responsive On switch mRNA, or (c) protein-responsive Off switch mRNA. The mRNA switch may be any one of (a), (b), and (c), or be two of them or all three of them. Furthermore, one, two, three, four, five, six, seven, eight or more types of mRNA switches having any configuration of (a), (b) or (c) may be used. In any case, a plurality of types of mRNAs, for example, having different sequences of miRNA target site or trigger protein target site and expressing different marker proteins in response to different miRNAs or trigger proteins, can be used.

### Introduction of mRNA switches

In the present invention, an mRNA switch can be introduced into a cell in the form of mRNA. For example, the mRNA switch may be introduced into a somatic cell by a technique such as lipofection or microinjection.

When two or more different mRNA switches are introduced, or when an mRNA switch and an mRNA to be used as a control (hereinafter also referred to as control mRNA) are introduced, it is preferred that a plurality of mRNAs be co-introduced into a cell group. Since the percentage of two or more co-introduced mRNAs in the cells is maintained in individual cells, the activity ratio of the proteins expressed from these mRNAs is constant within the cell group. The amount of introduction at this time varies depending on the cell group into which it is to be introduced, the mRNA to be introduced, the method of introduction, and the type of introduction reagent, and one skilled in the art is able to select these as appropriate to obtain the desired amount of translated markers. In the present invention, the control mRNA refers to an mRNA that does not have a target site of the miRNA or trigger protein. That is, the control mRNA is an mRNA that is introduced into the cells and translated without being affected by the expression level of endogenous miRNA or trigger protein. In a preferred embodiment, the control mRNA is introduced into a cell group together with the mRNA switch, and can function as a control for confirming and distinguishing the cell into which the mRNA switch has been introduced. The amount of the control mRNA introduced can also be suitably selected by one skilled in the art to obtain the desired amount translated.

When an mRNA switch is introduced into a cell, depending on each configuration feature of (a) miRNA-responsive Off switch mRNA, (b) miRNA-responsive On switch mRNA, or (c) protein-responsive Off switch mRNA, the translation of the marker protein is regulated in response to the miRNA or the protein present in the cells. On the other hand, the control mRNA is translated regardless of the presence or absence of the substances in the cells.

The step of distinguishing the cells (hereinafter also referred to as the distinguishing step) is then performed using the translation amount of the marker protein as an indicator. In the distinguishing step, cells are distinguished based on the translation amount of the marker protein, as described above. For example, cells with low level or high-level expression of miRNA serving as an indicator can be determined by obtaining a ratio of the translation amount of the marker protein between the cells belonging to a cell group including two or more types of cells. When a cell killing protein is used as a marker protein, cell death can be specifically induced depending on the expression level of miRNA serving as an indicator and the cell is removed.

The distinguishing step can be performed by detecting a signal from the marker protein using a predetermined detection device. Examples of the detection device include, but are not limited to, a flow cytometer, an imaging cytometer, a fluorescence microscope, a light emission microscope, and a CCD camera. Such detection devices can be appropriately employed by one skilled in the art, depending on the marker protein and the distinguishing method. For example, when the marker protein is a fluorescent protein or a luminescent protein, the marker protein can be quantified using a detection device such as a flow cytometer, an imaging cytometer, a fluorescence microscope, or a CCD camera. When the marker protein is a protein that assists fluorescence, luminescence, or coloration, a quantification method of the marker protein using a detection device such as a light emission microscope, a CCD camera or a luminometer can be used. When the marker protein is a membrane-localized protein, a quantification method of the marker protein using a cell surface protein-specific detection reagent such as an antibody and a detection device as described above, as well as a cell isolation method that does not require the marker protein quantification process, such as a method using a magnetic cell separator (MACS), can be used. When the marker protein is a drug resistance gene, a method for isolating a viable cell by detecting the expression of the marker protein with drug administration can be used.

Preferred examples of the detection method when the marker protein is a fluorescent protein include flow cytometry. Flow cytometry can provide the intensity of light emitted from fluorescent proteins or luminescent enzymes, which are marker proteins translated in individual cells, as distinguish information.

According to the present invention, the expression level of the marker protein can be increased, for example, by 3 to 25 times compared with the prior art. In particular, the present invention can express membrane proteins and secretory proteins, which could hardly be expressed in the prior art even by using an mRNA switch containing no N¹-methyl pseudouridine, to such an extent that cell distinguish is sufficiently performed. Thereby, using the switch of the present invention, it is possible, for example, to express the alkaline phosphatase in the cell membrane of a specific cell, and remove the specific cell with the D-3 peptide (WO2016/114341) which is a kind of phospho-D peptide; and to express a specific membrane protein only in a target cell and select the target cell with an antibody or a bead.

### [Examples]

Hereinafter, the present invention is described with Examples in more detail. However, the following Examples are not for limiting the present invention. The mRNA switch used in the Examples is a miRNA-responsive off switch mRNA or a protein-responsive off switch mRNA, in which the translation of a marker protein is suppressed in response to a specific miRNA or protein. In the following notation, "off switch" is omitted. In addition, the notation "miRNAXXX-responsive YYY mNRA" refers to an mRNA having a miRNAXXX target sequence and encoding YYY as a marker protein.

### Construction of DNA templates

The primers used to generate all mRNAs in the present example are shown in Table 5. The EGFP open reading frame was amplified from plasmid pFr15-EGFP, pCTp-EGFP or pAptamerCassette-EGFP using a primer set of TAP_IVTrev and TAP EGFP_IVT fwd or a primer set of KWC00405_EGFP mRNA1 ORF_Fw and KWC00427_EGFP ORF_Rv. The BFP open reading frame was amplified from plasmid pcDNA3.1-tagBFP using a primer set of TAP_IV Trev and TAP tagBFP_IVT fwd. The iRFP670 open reading frame was amplified from plasmid pC9-NirFP using a primer set of TAP_IV Trev and iRFP670 orf fwd. The hmAG open reading frame was amplified from plasmid pCTp-hmAG1-M9 or pFucci-S/G2/M Green using a primer set of hmAG1_IVT fwd and hmAG1_IV Trev. The MS2CP open reading frame was amplified from plasmid pTAP-MS2CP using a primer set of TAP MS2CP_IVTfwd and TAP_IVTrev. The Lin28 open reading frame was amplified from plasmid pLIN28 Amyc-T2A-tagRFP using a primer set of Lin28_Tfwd and Lin28_Trev. The L7Ae open reading frame was amplified from plasmid pTAP-L7Ae using a primer set of L7Ae_IVTfwd and L7AeRev. The U1A open reading frame was amplified from plasmid pU1Afullmyc-T2A-tagRFP using a primer set of KWC00430_U1 Afull ORF_Fw and KWC00431_U1 Afull ORF_Rv. The open reading frame of alkaline phosphatase (PALP) was amplified from plasmid OHU18818 using a primer set of 5'AL Porf and 3'AL Porf new. This alkaline phosphatase is a human placental alkaline phosphatase containing a GPI fragment. The open reading frame of SEAP was amplified from plasmid OHU 18818 using a primer set of 5' AL Porf and ALPP_rev_2. For the generation of pDisplay-ALP, first, the catalytic domain of ALP was amplified from plasmid 18818 using a primer set of ALPP_ORF_fwd and ALPP_ORF_rev. The pDisplayALP is a human placental alkaline phosphatase in which the GPI fragment is removed and a pDisplay fragment is added. The 5' Igk signal peptide sequence was amplified in advance from pDisplay plasmid (Invitrogen) using a primer set of Signal Peptide_IVT_fwd and Signal Peptide_IVT_rev. The 3' PDGFR transmembrane domain was amplified from the same pDisplay plasmid using a primer set of PDGFR_IVT_fwd and PDGFR_IVT_rev. The secondary structure of the truncated ALPP peptide, especially the catalytic residues was predicted to strictly match the known structure of wild-type ALPP determined by Phyre² online software (http://www.sbg.bio.ic.ac.uk/phyre2/html/page.cgi?id=index). The full-length pDisplay-AlPorf was generated by fusion PCR using three fragments and a primer set of Signal Peptide_IVT_fwd and PDGFR_IVT_rev.

**[Table 5]**

| **Primer** | **Sequence 5' to 3'** | SEQ ID NO: |
|---|---|---|
| TAP_IVTrev | GCCCCGCAGAAGGTCTAGAtcactcgagatgcatatgagatc | 6 |
| TAPEGFP_IVTfwd | CACCGGTCGCCACCATGGGATCCGTGAGCAAGGGC | 7 |
| TAPtagBFP_IVTfwd | CACCGGTCGCCACCATGGGATCCAGCGAG | 8 |
| TAPiRFP_IVT fwd | CACCGGTCGCCACCATGGCGCGTAAGGTCGATCTC | 9 |
| TAPiRFP_IVT rev | GCCCCGCAGAAGGTCTAGATCACTCGAGATGCatatgagatc | 10 |
| hmAG1_IVT fwd | CACCGGTCGCCACCATGGTGAGCGTGATCAAGCCCG | 11 |
| hmAG1_IVT rev | GCCCCGCAGAAGGTCTAGATTCACTTGGCCTGGCTGGGC | 1 2 |
| TAPMS2CP_IVTfwd | CACCGGTCGCCACCATGGGATCCGCTTC | 13 |
| Lin28_Tfwd | GCCACCATGGGATCCGGCTCCGTGTCCAAC | 14 |
| Lin28_Trev | TATGAGATCTATTCTGTGCCTCCGGGAG | 1 5 |
| L7Ae_IVTfwd | CACCGGTCGCCACCATGTACGTGAGATTTGAGGTTCCTG | 16 |
| L7Ae Rev primer | GGGGGgaattCTCACTTCTGAAGGCCTTTAATCTTCTCC | 1 7 |
| 5' ALP orf | caccggtcgccaccatgCTGGGGCCCTGCATGCTGCTg | 18 |
| 3' ALP orf new | gccccgcagaaggtctagactaGGGAGCAGTGGCCGTCTCCAGCA | 19 |
| ALPP_rev_2 | GCCCCGCAGAAGGTCTAGACTAgtcggtggtgccggcgg | 20 |
| ALP_ORF_fwd | GCCCAGCCGGCCAGATCTatcatcccagttgaggaggagaaccc | 21 |
| ALP_ORF_rev | agaaccaccaccagaaccaccaccgtcggtggtgccggcgg | 22 |
| SignalPeptide_IVT_fwd | CACCGGTCGCCACCATGGAGACAGACACACTCCTGCTATGG | 23 |
| SignalPeptide_IVT_rev | AGATCTGGCCGGCTGGGC | 24 |
| PDGFR_IVT_fwd | ggtggtggttctggtggtggttctGAACAAAAACTCATCTCAGAAGAG GATCTGAATGC | 25 |
| PDGFR_IVT_rev | GCCCCGCAGAAGGTCTAGACTACTAACGTGGCTTCTTCTGCCA AAGCATG | 26 |
| ORF_puroR_fwd | CACCGGTCGCCACCATGACCGAGTACAAGCCCACG | 51 |
| ORF_puroR_rev | GCCCCGCAGAAGGTCTAGATCAGGCACCGGGCTTGC | 52 |
| KWC00430_U1Afull ORF_Fw | CACCGGTCGCCACCATGGcgGCAGTTCCCGAGACCCG | 57 |
| KWC00431_U1Afull ORF_Rv | GCCCCGCAGAAGGTCTAGACTACTTCTTGGCAAAGGAGATCTT CATG | 58 |
| KWC00405_EGFP mRNA 1 ORF_Fw | CACCGGTCGCCACCATGGTGAGCAAGGGCGAG | 59 |
| KWC00427_EGFP ORF_Rv | GCCCCGCAGAAGGTCTAGACTACTTGTACAGCTCGTCCATGCC GAGAG | 60 |

The 3' UTR fragment was amplified by PCR from template IVT_3 prime_UTR using a primer set of Fwd3UTR and Rev3UTR2T20. For control mRNAs having no miRNA target sites, 5' UTR fragments were amplified by PCR from template IVT_5prime_UTR using a primer set of TAP_T73GC and Rev5UTR. For the 5UTR of switch mRNAs having miRNA target sites, each sequence listed in Table 6 below was used.

**[Table 6]**

| **miRNA** | | **5UTR secuence for miR switch** | | SEQ ID NO: |
|---|---|---|---|---|
| Name | ID | Name | Sequence 5' **to** 3' | |
| hsa-miR-661 | MI0003669 | 5UTRtemp_T661 | CGACTCACTATAGGTTCCGCGATCGCGGATCCACGCGC AGGCCAGAGACCCAGGCAAGATCACCGGTCGCCACCAT G | 27 |
| hsa-miR-335 | MIMAT0000765 | 5UTRtemp_T335-5p | CGACTCACTATAGGTTCCGCGATCGCGGATCCACATTT TTCGTTATTGCTCTTGAAGATCCACCGGTCGCCACCATG | 28 |
| hsa-miR-210 | MIMAT0000267 | 5UTRtemp_T210 | CGACTCACTATAGGTTCCGCGATCGCGGATCCTCAGCC GCTGTCACACGCACAGAGATCACACCGGTCGCCACCAT G | 29 |
| hsa-miR-21-5P | MI0000077 | 5UTRtemp_T21-5p | CGACTCACTATAGGTTCCGCGATCGCGGATCCTCAACA TCAGTCTGATAAGCTAAGATCACACCGGTCGCCACCAT G | 30 |
| hsa-miR-21-5p | MI0000077 | 5UTRtemp_T4x21-5p | CGACTCACTATAGGTTCCGCGATCGCGGATCCtcaacatc agtctgataagctatcaacatcagtctgataagctatcaacatcagt ctgataagctatcaacatcagtctgataagctaAGATCACACCG GTCGCCACCATG | 31 |
| hsa-miR-302a | MIMAT0000684 | 5UTRtemp_T302a-5p | CGACTCACTATAGGTTCCGCGATCGCGGATCCAGCAAG TACATCCACGTTTAAGTAGATCCACCGGTCGCCACCATG | 32 |
| hsa-mi R-302a | MIMAT0000684 | 5UTRtemp_4×302a-5p | CGACTCACTATAGGTTCCGCGATCGCGGATCCagcaagt acatccacgtttaagtagcaagtacatccacgtttaagtagcaagta catccacgtttaagtagcaagtacatccacgtttaagtAGATCACA CCGGTCGCCACCATG | 33 |
| hsa-mi R- 17-5p | | 5UTR_17-5p | ggucagauccgcuaggaucccuaccugcacuguaagcacuuug agaucuaccggucgccaccaug | 34 |
| hsa-miR-206 | | 5UTR_206 | gguuccuuaaucgcggaucccccacacacuuccuuacauuccaag aucacaccggucgccaccaug | 35 |
| hsa-miR-92a | | 5UTR-92a | cgacucacuauaggucagauccgcuaggauccacaggccgggac aagugcaauaagaucuaccggucgccaccaug | 36 |

For the 5UTR of protein-responsive switch mRNAs, each sequence listed in Table 7 below was used. For the Lin28-responsive mRNA, two primers, stbC Fwd primer and stbC spacer, were used as 5' UTR sequences in fusion PCR. TAP_T73GC was not used in this reaction. For the MS2CP-responsive mRNA, the 5' UTR was obtained from template plasmid pscMS2 (WTx2)-EGFP using a primer set of GCT7CMV_G and Rev5UTR. For L7Ae-responsive mRNA, two primers, KWC0131_T7-kt-EGFP_Fw and Spacer 5UTR Fwd, were used as 5UTR sequences in fusion PCR. The TAP_T73GC was not used in this reaction. The U1A response switch was prepared by fusion PCR using three primers, KWC00433_T7-Ulutr-EGFP_v2_fw, KWC00434_T7+g_Fw, KWC0137_Spacer5UTR_ver2, and a fluorescent reporter open reading frame, 3' UTR fragment, and 3UTR120A.

**[Table 7]**

| **RNA binding protein** | **RNA motif** | **primers used to obtain 5 UTR sequence** | | SEQ ID NO: |
|---|---|---|---|---|
| | | **Name** | **Sequence** 5' **to** 3' | |
| MS2CP | MS2 | GCT7CMV_G | GCTAATACGACTCACTATAGGTCAGATCCGCTAGCG | 37 |
| | | Rev5UTRf or MS2 | CATGGTGGCGACCGGTggg | 38 |
| LIn28 | stbC | stbC Fwd primer | GAAATTAATACGACTCACTATAgggggcTTAGGGCgGGGA TTTcGCCCACAAG GAGGTgccccGGACACCCGCAGA TC G | 39 |
| | | stbC spacer | ACACCCGCAGATCGAGAAGAAGGCGAATTAAGAGAGAAA AGAAGAGTAA GAAGAAATATAAGACACCGGTCGCCACCA TG | 40 |
| L7Ae | kink turn | KWC0131_T7-kt-EGFP_Fw | GAAATTAATACGACTCACTATAGGATCCGTGATCGGAAA CGTGAGATCCACCTCAGATCCGCTAGGACACCCGCAGAT CG | 41 |
| | | Spacer5UTRFwd | ACACCCGCAGATCGAGAAGAAGGCGAATTAAGAGAGAAA AGAAGAGTAAGAAGAAATATAAGACACCGGTCGCCACCA TG | 42 |
| U1A | U1 sn V2 | T7-U1utr-EGFP_v2_fw | TAATACGACTCACTATAgACAGCATTGTACCCAGAGTCTG TCCCCAGACATTGCACCTGGCGCTGTCCGCAGATCGAGA AGAAGGCGAATTAAGAGAGAAAAGAAG | 53 |
| | | T7+g_Fw | CAGTGAATTgTAATACGACTCACTATAg | 54 |
| | | KWC0137_Spacer5UTR_ver2 | AGATCGAGAAGAAGGCGAATTAAGAGAGAAAAGAAGAGT AAGAAGAAATATAAGACACCGGTCGCCACCATG | 61 |

The IVT DNA template for mRNA synthesis was amplified by fusion PCR. For all mRNA switches, the protein open reading frame fragment (10 ng), 5' UTR sequence (10 nM), and 3' UTR sequence (10 nM) were used, and a primer set of GCT7pro_5UTR and 3UTR120A was used. For control mRNAs that were not switches, a primer set of TAP_T73GC and 3UTR120A was used unless otherwise specified. Primers used for fusion PCR are shown in Table 8.

**[Table 8]**

| | Primers used to generate full DNA templates and UTR sequen ces | SEQ ID NO: |
|---|---|---|
| **Primer** | **Sequence** 5' **to** 3' | |
| GCT7pro_5UTR | GCTAATACGACTCACTATAGGTTCCTTAATCGCGGATCC | 43 |
| 3UTR120A | TTTTTTTTTTTTTTTTTTTTTTTTTTTTTTTTTTTTTTTTTTTTTTTTTTTTTTTTT TTTTTTTTTTTTTTTTTTTTTTTTTTTTTTTTTTTTTTTTTTTTTTTTTTTTTTTTT TTTTTTTTTTTTTTTTTTTTTTTTTTTTCCTACTCAGGCTTTATTCA | 44 |
| Fwd3UTR | TCTAGACCTTCTGCGGGGC | 45 |
| IVT_3prime_UTR | TCTAGACCTTCTGCGGGGCTTGCCTTCTGGCCATGCCCTTCTTCTCT CCCTTGCACCTGTACCTCTTGGTCTTTGAATAAAGCCTGAGTAGG | 46 |
| Rev3UTR2T20 | TTTTTTTTTTTTTTTTTTTTCCTACTCAGGCTTTATTCAAAGACCAAG | 47 |
| TAP_T73GC | CAGTGAATTGTAATACGACTCACTATAGGGC | 48 |
| IVT_5prime_UTR | CAGTGAATTGTAATACGACTCACTATAGGGCGAATTAAGAGAGAAAA GAAGAGTAAGAAGAAATATAAGACACCGGTCGCCACCATG | 49 |
| Rev5UTR | CATGGTGGCGACCGGTGTCTTATATTTCTTCTTACTC | 50 |

### In vitro Transcription

All mRNAs were prepared using Mega Script T7kit (Ambion) according to the protocol. In this reaction, for the synthesis of Ψ-mRNA, pseudouridine-5'-triphosphate was used instead of uridine triphosphate. For the synthesis of m1Ψ-mRNA, N¹-methyl pseudouridine-5'-triphosphate (N¹-methyl-pseudouridine-5'-triphosphate) was used instead of uridine triphosphate. For the synthesis of m5C-mRNA, 5-methylcytidine-5'-triphosphate was used instead of cytidine triphosphate. For the synthesis of m5C/m1Ψ-mRNA, pseudouridine-5'-triphosphate and 5-methylcytidine-5'-triphosphate were used instead of uridine triphosphate and cytidine triphosphate. For the synthesis of m5C/m1Ψ-mRNA, N¹-methyl pseudouridine-5'-triphosphate and 5-methylcytidine-5'-triphosphate were used instead of uridine triphosphate and cytidine triphosphate. For the synthesis of m6An mRNA, N6-methyladenosine-5'-triphosphate was used instead of adenosine triphosphate. For the synthesis of m6A/m1Ψ-mRNA, N¹-methyl pseudouridine-5'-triphosphate and N6-methyladenosine-5'-triphosphate were used instead of uridine triphosphate and adenosine triphosphate. All modified nucleic acids were obtained from TriLink BioTechnologies, Inc. It should be noted that nucleic acids denoted as mRNA "composed of modified bases" in the Examples were those in which all applicable bases consisted of modified bases.

Prior to the IVT reaction (mRNA synthesis), guanosine triphosphate was diluted 5-fold with Anti Reverse Cap Analog (New England Biolabs Inc.). The reaction mixture was incubated at 37°C for 4 hours, and to the resulting product, TURBO DNase (Ambion) was added. The obtained mixture was incubated at 37°C for an additional 30 minutes. The resulting mRNA was purified with RNeasy Mini Elute Cleanup Kit (Qiagen), was then incubated with Antarctic Phosphatase (New England Biolabs Inc.) at 37°C for 30 minutes, and was further purified with RNeasy Mini Elute Cleanup Kit (Qiagen). The prepared mRNA was determined by A260 reading on Nanodrop (Thermo Fisher Scientific). The purity of the mRNA was determined by Bio Analyzer (Agilent Technologies, Inc.) using 100 ng of mRNA with an RNA kit (Shimadzu Corporation) or RNA6000 Nano kit (Agilent Technologies, Inc.). The quality-checked mRNA was then diluted to 100 ng/µL and stored at -30°C until use.

### Cell culturing

All cells were cultured under the conditions of 37°C and 5% CO₂. HeLa cells (ATCC) were cultured in high glucose Dulbecco's modified Eagle medium DMEM (NACALAI TESQUE, INC.) supplemented with 10% fetal bovine serum (FBS), 1 mM sodium pyruvate (Invitrogen), and 0.1 mM non-essential amino acids (NEAA) (Invitrogen). HeLa cells stably overexpressing EGFP were cultured in the same medium as described above. The 293FT cells (Invitrogen) were cultured with high glucose DMEM containing 10% FBS, 0.1 mM NEAA, and 1 mM sodium pyruvate. Normal human skin fibroblasts (NHDF; Lonza) were cultured in complete fibroblast basal medium. Human ovarian cancer cells A2780 (Sigma-Aldrich Co. LLC.) were cultured in 1640 RPMI medium supplemented with 10% FBS and 2 mML-glutamine. SH-5YSY cells (Sigma-Aldrich Co. LLC.) were cultured in DMEM-F12 (1:1) supplemented with 10% FBS, 2 mML-glutamine, and 0.1 mM NEAA. The human feeder-free hiPSC strains 201B7 (Ff-201B7) were provided by Dr. Makoto Nakagawa of Kyoto University and the cell preparation facility of the Center for iPS Cell Research and Application, Kyoto University, and were maintained with iMatrix-511 (Nippi, Inc.) in complete Stem Fit (AK03) medium (AJINOMOTO CO., INC.). Naturally differentiated cells were obtained based on Ff-201B7 described above by removing basal growth factors from the Stem Fit medium and culturing for 2 weeks.

### Transfection

The cultured cells were placed in a 24-well plate at 0.5 x 10⁵ cells/well. The following day, mRNA was introduced into the cells. The introduction was performed with 1 µL of Lipofectamine MessengerMax (Thermo Fisher Scientific), according to the manufacturer's instructions. For measurement of mRNA expression, mRNAs (200 ng) each consisting of a nucleotide sequence having a different modified base were introduced into each cell. For expression of cytoplasmic fluorescent proteins, the cells were measured by flow cytometry with AccuriC6 (BD Biosciences) 24 hours after the introduction. The mean expression level of EGFP in viable cells (7-AAD-negative cells) was measured and compared to that in cells transfected with native mRNA that has no modified bases. The percentage of viable cells (ratio of 7-AAD negative cells to all cells) and the percentage of transfection (ratio of EGFP positive cells to all viable cells) were also measured with AccuriC6. For expression of membrane proteins, lysates of each sample by Cell Lytic-M (Sigma-Aldrich Co. LLC.) were collected 24 hours after the introduction. The total protein mass of each sample was measured with BCA Protein Assay Kit (Pierce), and the alkaline phosphatase activity of each sample was measured with an alkaline phosphatase detection kit Fluorescence (Sigma-Aldrich Co. LLC.) using the same amount of total protein. The relative expression level of the protein was determined by comparing with the expression of cells transfected with native mRNA. For the secretory protein SEAP, the alkaline phosphatase activity in the culture medium of each sample was measured with an alkaline phosphatase detection kit Fluorescence (Sigma-Aldrich Co. LLC.) using the same amount of medium. The relative expression level of the protein was determined by comparing with the expression of cells transfected with native mRNA.

In the experiments using miRNA-responsive mRNA, 100 ng of miRNA-responsive mRNA were co-introduced with 90 ng of control mRNA. The control mRNA used was one composed of m5C/Ψ modified bases. The cells were measured by flow cytometry with Accuri C6 (BD Biosciences) 24 hours after the introduction. The mean fluorescence intensity of marker proteins encoded by the miRNA-responsive mRNAs in each cell was normalized by the mean intensity of control mRNA. The dynamic range was determined by dividing the intensity of the normalized marker protein in the sample not containing miRNA by the intensity of the normalized marker protein in the sample containing the corresponding miRNA. The dynamic range and Fold Change described later are used interchangeably.

In the miRNA mimic assay, 3 to 0.0003 nM miRNA mimics were co-introduced into 293FT cells with 90 ng of miRNA-responsive mRNA and 90 ng of control mRNA. The cells were measured by flow cytometry with AccuriC6 or Aria (BD Biosciences) 24 hours after the introduction. The mean fluorescence intensity of marker proteins encoded by the miRNA-responsive mRNAs in each cell was normalized by the mean intensity of control mRNA. The percentage of the suppression by the miRNA mimic was calculated by comparing the mean fluorescence intensity of the normalized marker protein in each sample with the mean fluorescence intensity of the normalized marker protein in the sample not containing the miRNA mimic.

In the experiments using protein-responsive mRNA, 200 ng of protein-responsive mRNA and 90 ng of control mRNA were co-introduced into cells with or without the mRNA encoding the trigger protein. The mRNA encoding a trigger protein is an mRNA encoding a trigger protein that specifically binds to the protein-responsive mRNA. When the trigger protein was L7Ae, 30 ng of L7Ae mRNA (mRNA encoding L7Ae) was co-introduced. When the trigger protein was MS2 or Lin28, 100 ng of MS2 mRNA (mRNA encoding MS2) or Lin28 mRNA (mRNA encoding Lin28) was co-introduced. Cells were measured by flow cytometry with Accuri C6 24 hours after the introduction. The mean fluorescence intensity of marker proteins encoded by the protein-responsive mRNA in each cell was normalized by the mean intensity of control mRNA. The dynamic range was determined by dividing the intensity of the normalized marker protein in the sample not containing miRNA encoding the trigger protein by the intensity of the normalized marker protein in the sample containing the corresponding miRNA encoding the trigger protein.

### Flow cytometry

24 hours after the transfection, cells were separated from the culture dish and passed through the mesh and analyzed by flow cytometry. The hmAG1 and EGFP were detected with a blue laser equipped with a 90% cut FITC filter (530/30 nm) with a wavelength of 488 nm. The tagBFP was detected with a purple laser equipped with Pacific Blue filter (450/40 nm) with a wavelength of 405 nm. The iRFP670 was detected with a red laser and a violet laser each equipped with a BP filter (675/25 nm) with a wavelength of 633 nm. Dead cells and debris were removed by forward and backward light scatter signals and were further determined by 7-AAD staining.

### Time-lapse imaging

Immediately after the transfection, HeLa cells that stably expressed EGFP were placed on Cell aview (KELLER MSR), and cultured in the dark at 37°C and 5% CO₂. The fluorescence intensities of four different areas of each cell were recorded every hour. The fluorescence intensity of each sample was obtained by averaging with the fluorescence intensity of iRFP670 and then normalizing by the averaged intensity of endogenous EGFP.

### Pathscan(R) Assay

6 hours after transfection, cell lysates were collected according to the pathscan assay protocol (Cell Signaling Technology). According to the same protocol, 10 µg/mL poly IC as a positive control was introduced into the cells with Lipofectamine MessengerMax. As a negative control, these cells were treated only with the transfection reagent Lipofectamine MessengerMax.

### In vitro Translation

*In vitro* translation was performed in rabbit reticulocytelysate (Ambion) in a flat black half-area 96-well solid plate, according to the protocol. For the rabbit reticulocytelysate assay, 25 µl of reactant was added to 300 ng of EGFP mRNA. Fluorescence of EGFP was measured every 10 minutes for 12 hours at 37°C by TECAN plate-reader from the top.

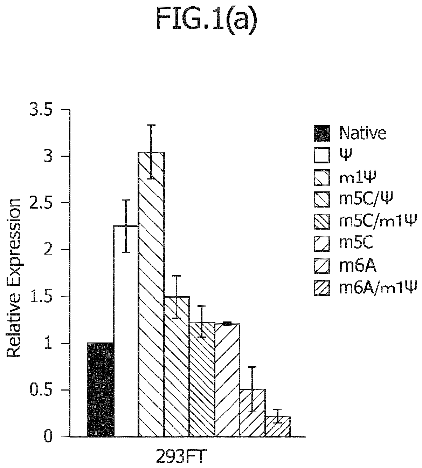

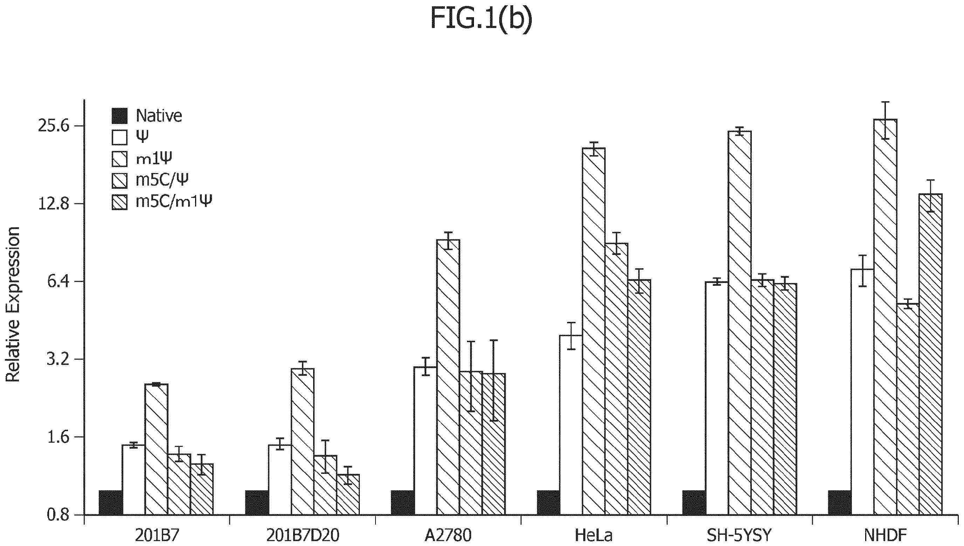

Figs. 1(a) and 1(b) are graphs showing the results of expressing EGFP mRNAs each consisting of a nucleotide sequence having a varied modified base in different cells. EGFP mRNA is a control mRNA that has neither miRNA target sites nor trigger protein target sites. The cells were placed into 0.5 mL of medium in a 24-well plate at 0.5 x 10⁵ cells. 24 hours after the placing, transfection was performed by dissolving 200 ng of mRNA and 1 µL of Lipofectamine MessengerMax in 50 µL of Opti MEM. The expression of EGFP was measured 24 hours after the transfection by an Accuri flowcytometer (BD Bioscience) with a 90% cutFL-1 filter. In the graph, Native represents EGFP mRNA consisting of a nucleotide sequence having no modified bases. Ψ, m1Ψ, m5C/Ψ, m5C/m1Ψ, m5C, m6A, and m6A/m1Ψ are mRNAs each consisting of a nucleotide sequence having a modified base(s) described in the *"In vitro* Transcription" compared with the native mRNA.

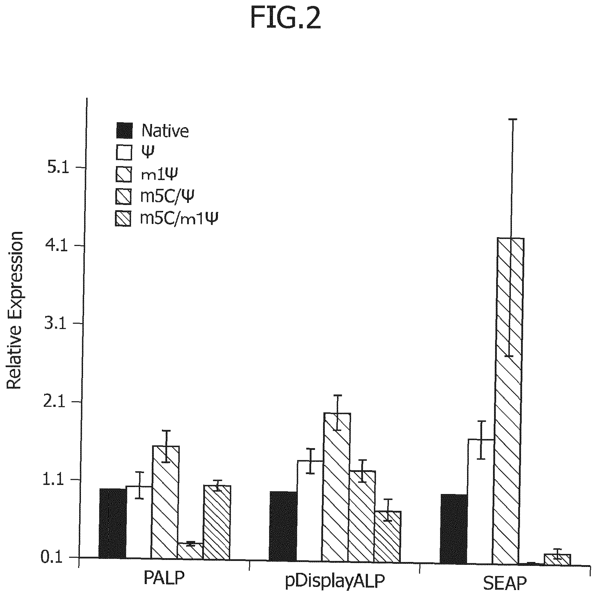

Fig. 2 is a graph showing the results of expression of three types of alkaline phosphatase mRNAs each consisting of a nucleotide sequence having a varied modified base in cells. These mRNAs are control mRNAs that have neither miRNA target sites nor trigger protein target sites. The cells were placed into 0.5 mL of medium in a 24-well plate at 0.5 x 10⁵ cells. 24 hours after the placing, transfection was performed by dissolving 200 ng of mRNA and 1 µL of Lipofectamine MessengerMax in 50 µL of Opti MEM. The expression of alkaline phosphatase was measured with Alkaline Phosphatase Detection Kit by measuring fluorescence 24 hours after the transfection. The expression was adjusted based on the total protein mass. In the graph, PALP refers to a protein expressed based on mRNA encoding human placental alkaline phosphatase containing a GPI fragment, pDisplay-ALP refers to a protein expressed based on mRNA encoding human placental alkaline phosphatase in which the GPI fragment is removed and a pDisplay fragment is added, and SEAP refers to a protein expressed based on mRNA encoding human placental alkaline phosphatase without GPI fragments, respectively. PALP and pDisplay-ALP were measured in the cell lysates, and SEAP expression was measured in the culture medium. The expression from cells in cell lysates or culture medium transfected with Lipofectamin MessengerMax alone was used as a blank for this experiment. In the graph, Native represents alkaline phosphatase mRNA consisting of a nucleotide sequence having no modified bases. Ψ, m1Ψ, m5C/Ψ, and m5C/m1Ψ are mRNAs each consisting of a nucleotide sequence having a modified base(s) described in the *"In vitro* Transcription" compared with the Native mRNA.

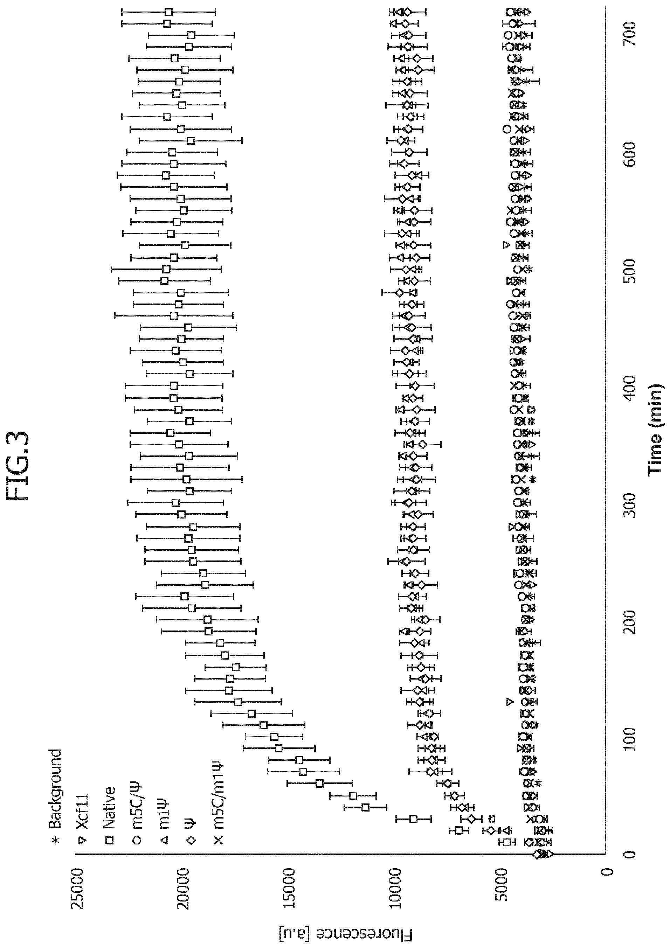

Fig. 3 is a graph showing the relationship between time and fluorescence intensity when *in vitro* translations of EGFP mRNAs each consisting of nucleotide sequences having a varied modified base were performed in Rabbit Reticulocyte Lysate. In the *in vitro* translation assay, 300 ng of each mRNA was added to Invitrogen Rabbit Reticulocyte Lysate. The fluorescence intensity was measured every 10 minutes with a TECAN plate-reader. In the graph, Native represents an mRNA consisting of a native nucleotide sequence containing no modified bases, and m5C/Ψ, m1Ψ, Ψ, and m5C/m1Ψ each represent an mRNA consisting of a nucleotide sequence having a modified base(s) described in the *"In vitro* Transcription". The term "Background" means a sample consisting of Rabbit Reticulocyte Lysate alone.

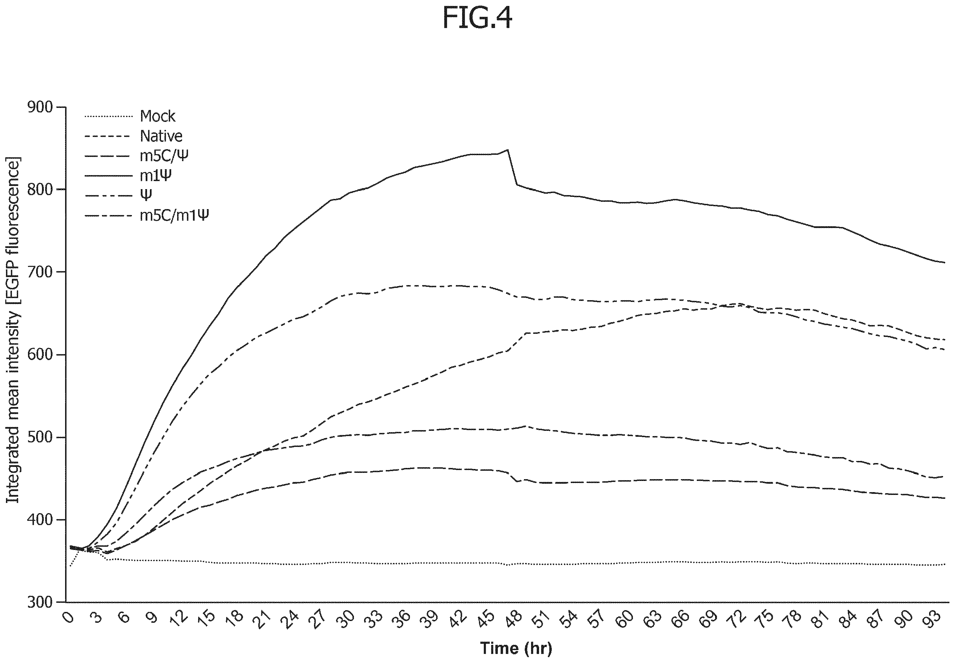

Fig. 4 is a graph showing the time-dependent expression of EGFP mRNA in 239FT cells. 239FT cells were placed into 0.5 mL of medium in a 24-well plate at 0.5 x 10⁵ cells. 24 hours after the placing, transfection was performed by dissolving 200 ng of mRNA and 1 µL of Lipofectamine MessengerMax in 50 µL of Opti MEM. Recording of EGFP expression was started immediately after the transfection and was measured by a cell image analysis system CELAVIEW (Olympus Corporation). The integrated mean intensity of EGFP channel was plotted against time (hr). In the graph, Native represents an mRNA consisting of a native nucleotide sequence having no modified bases, and m5C/Ψ, m1Ψ, Ψ, and m5C/m1Ψ each represent an mRNA consisting of a nucleotide sequence having a modified base(s) described in the *"In vitro* Transcription". "Mock" indicates cells transfected only with 1 µL of Lipofectamine MessengerMax.

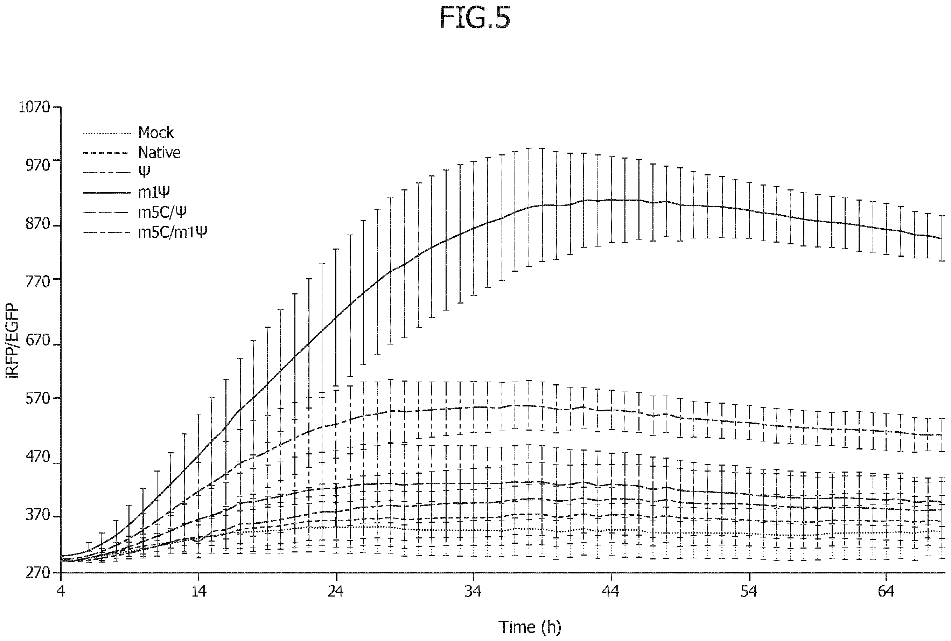

Fig. 5 is a graph showing the time-dependent expression of iRFP670 mRNA in HeLa cells that stably expressed EGFP (HeLa@EGFP). HeLa EGFP was placed into 0.5 mL of medium in a 24-well plate at 0.5 x 10⁵ cells. 24 hours after the placing, transfection was performed by dissolving 200 ng of iRFP670 mRNA and 1 µL of Lipofectamine MessengerMax in 50 µL of Opti MEM. Recording of EGFP expression was started 4 hours after the transfection and was measured by a cell image analysis system CELAVIEW (Olympus Corporation). The expression of iRFP670 was adjusted against endogenously expressed EGFP. The results of two trials are shown on average. Error bars indicate the standard error. In the graph, Native represents an mRNA consisting of a native nucleotide sequence having no modified bases, and m5C/Ψ, m1Ψ, Ψ, and m5C/m1Ψ each represent an mRNA consisting of a nucleotide sequence having a modified base(s) described in the "*In vitro* Transcription". "Mock" is defined in the same way as that in the result shown in Fig. 4.

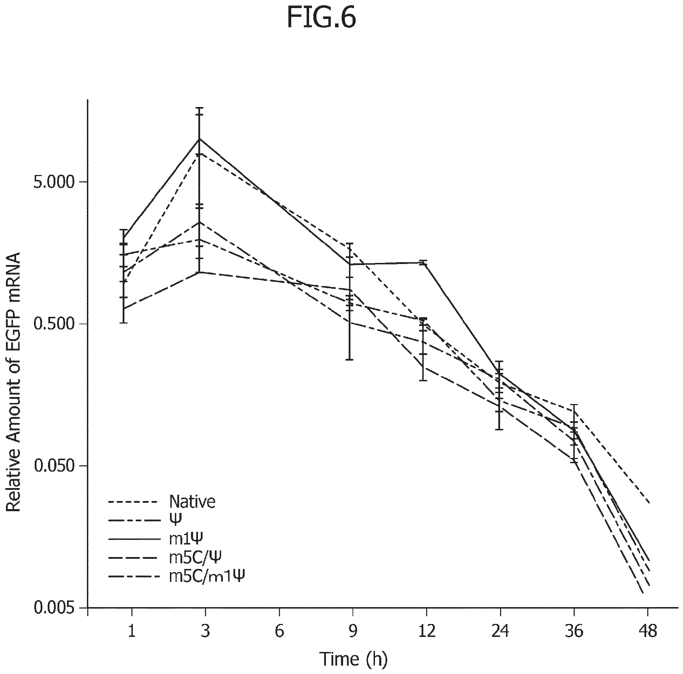

Fig. 6 shows time-dependent changes in the intracellular amount of EGFP mRNAs each consisting of a nucleotide sequence having a various modified base. 293FT cells were placed into 0.5 mL of medium in a 24-well plate at 0.5 x 10⁵ cells. 24 hours after the placing, transfection was performed by dissolving 200 ng of EGFP mRNA and 1 µL of Lipofectamine MessengerMax in 50 µL of Opti MEM. Cell lysates were collected at a predetermined time after the transfection, and then total RNA was extracted. The amount of EGFP was quantified by qPCR. In the graph, the amount of EGFP was expressed for each EGFP mRNA as a relative amount when the amount at 1 hour from the transfection was 1. In the graph, Native represents an mRNA consisting of a native nucleotide sequence having no modified bases, and m5C/Ψ, m1Ψ, Ψ, and m5C/m1Ψ each represent an mRNA consisting of a nucleotide sequence having a modified base(s) described in the *"In vitro* Transcription".

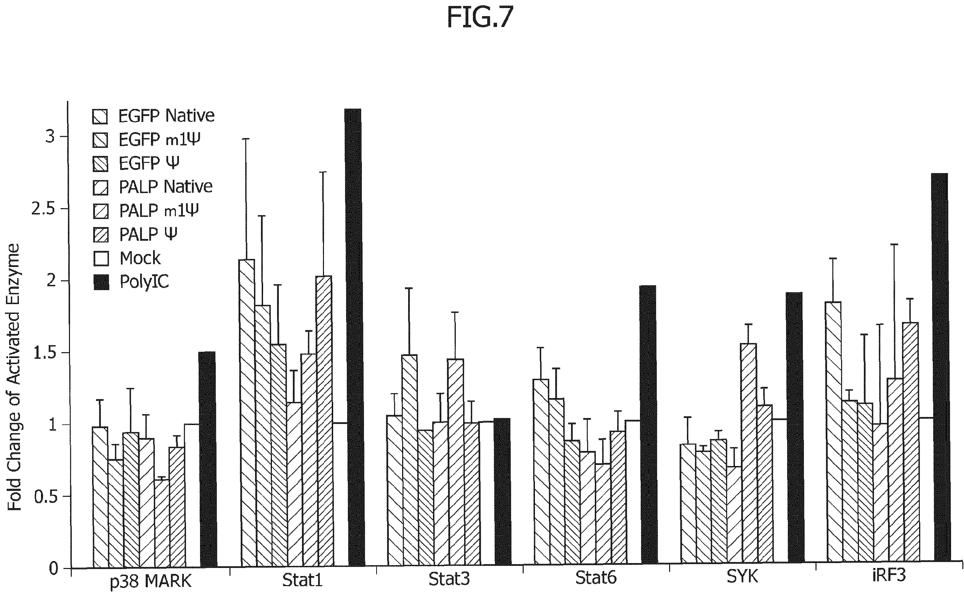

Fig. 7 shows the amount of active immune response marker 6 hours after the transfection in 293FT to which EGFP mRNAs and PALP mRNAs, each consisting of a nucleotide sequence containing a varied modified base, were transfected. The 293FT cells were placed into 0.5 mL of medium in a 24-well plate at 0.5 x 10⁵ cells. 24 hours after the placing, transfection was performed by dissolving 200 ng of each mRNA and 10 µg/mL polyIC, and 1 µL of Lipofectamine MessengerMax in 50 µL of OptiMEM. 6 hours after the transfection, cell lysate of each sample was collected according to the protocol of Pathscan(R) Immune Cell Signaling Antibody Array Kit (Fluorescent Readout). The amount of active immune response marker was measured with Pathscan Immune Cell Signaling Antibody Array Kit. In the graph, Native represents an mRNA consisting of a native nucleotide sequence having no modified bases, and m1Ψ and Ψ each represent an mRNA consisting of a nucleotide sequence having a modified base described in the *"In vitro* Transcription". "Mock" indicates cells transfected only with 1 µL of Lipofectamine MessengerMax.

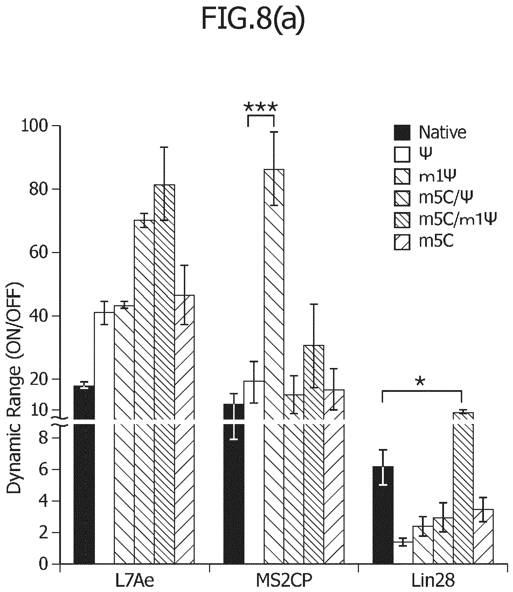

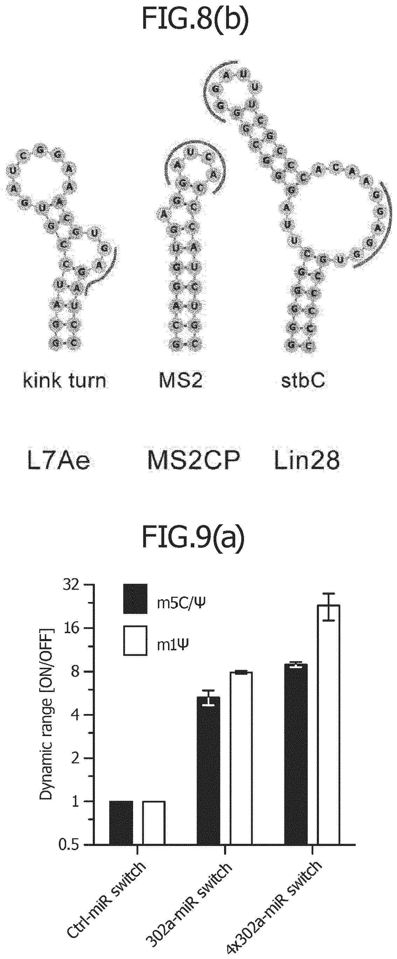

Fig. 8(a) is a graph showing the dynamic range of protein-responsive EGFP or hmAG1 mRNAs composed of nucleotides with or without a modified base. Fig. 8(b) shows a schema of the secondary structure of RNA-protein binding motifs and combinations with a trigger protein specifically binding thereto. The solid lines shown along the sequences means protein binding sequences. As the trigger proteins, L7Ae, MS2CP, and Lin28 were used, and as the sequence specifically recognized by the trigger protein, each nucleic acid sequence of the RNA-protein binding motif corresponding to these was used. 293 cells were placed into 0.5 mL of medium in a 24-well plate at 0.5 x 10⁵ cells. 24 hours after the placing, transfection was performed by dissolving 200 ng of protein-responsive EGFP or hmAG1 mRNA and 100 ng of control iRFP670 mRNA and 1 µL of Lipofectamine MessengerMax in 50 µL of Opti MEM under the presence or absence of mRNA encoding the trigger protein. The iRFP670 mRNA and the mRNA encoding the trigger protein used were those composed of m5C/Ψ modified bases. The expression of EGFP or hmAG1 was adjusted by the expression of iRFP670. The dynamic range is defined by the expression level of EGFP or hmAG1 in the absence of the trigger protein relative to the expression level of EGFP or hmAG1 in the presence of the trigger protein. Since the protein binding ability depends on both the primary and secondary structures of RNA, modification of nucleotides affected each system differently. Since the protein binding sequences of kink-turn and stbC did not contain uridine, the modification of uridine had limited impact on these two systems. In both systems, the m5C/m1Ψ modification resulted in the highest dynamic range. This is believed to be due to the fact that m5C and m1Ψ jointly stabilized the stem structure of the RNA motif. All of the samples showing the largest dynamic range had a m1Ψ modification.

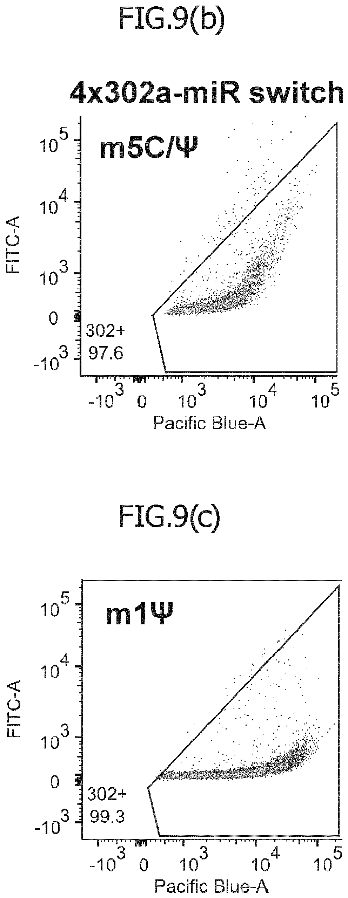

Figs. 9(a), 9(b), and 9(c) show that the miR-302a-responsive Off switch mRNA composed of nucleotides having a m1Ψ modified base has greater dynamic range than the miR-302a-responsive Off switch mRNA composed of nucleotides having m5C/Ψ modified bases. 90 ng of miR-302ahmAG mRNA and 180 ng of control tagBFP mRNA were transfected into 201B7 iPSCs with Lipofectamine MessengerMAX. As the miR-302a hmAG mRNA, one having a single miR-302a target sequence and one having 4 repeats of miR-302a target sequences were used. The Off switch mRNAs used were mRNAs composed of nucleotides having m5C/Ψ modified bases or m1Ψ modified base. The control tagBFP used was composed of m5C/Ψ modified bases. The dynamic range was calculated by dividing the translation efficiency (expression intensity of hmAG/expression intensity of tagBFP) in the switch-on state, i.e., in the case in which hmAG mRNA without a miRNA target sequence was used, by the translation efficiency in the switch-off state, i.e., in the case in which the miR-302a-responsive mRNA described above was used. From this result, it was found that an mRNA switch consisting of a nucleotide sequence having a m1Ψ modified base greatly improves the dynamic range of switch-on/off defined above.

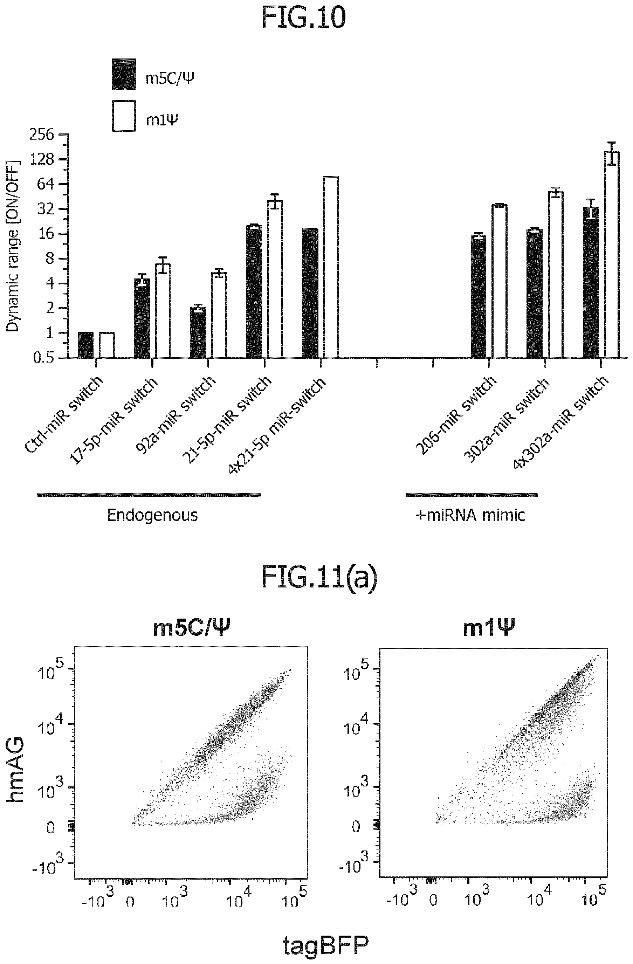

Fig. 10 shows that multiple types of miRNA-responsive Off switch mRNAs composed of nucleotides having a modified base improved the dynamic range in HeLa cells. 90 ng of miR-17-5p-responsive hmAG mRNA, miR-92a-responsive hmAG mRNA, miR-21-5p-responsive hmAG mRNA (miR-21-5p target sequences 1x and 4x), miR-206-responsive hmAG mRNA, or miR-302a-responsive hmAG mRNA (miR-302a target sequences 1x and 4x) and 180 ng of control tagBFP mRNA were transfected into HeLa cells with Lipofectamine MessengerMAX. For the miR-206-responsive hmAG mRNA and miR-302a-responsive hmAG mRNA, 4 pmol of the corresponding miRNA mimic was co-transfected. The Off switch mRNAs used were mRNAs composed of nucleotides having m5C/Ψ modified bases or m1Ψ modified base.

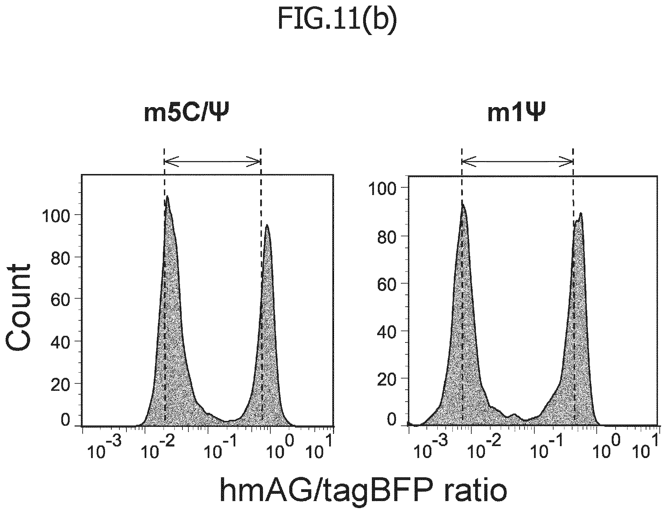

Fig. 11 shows that the spatial resolution of HeLa cells and 293FT cells is improved by using miR-21-5p-responsive mRNA composed of nucleotides having a m1Ψ modified bases. A 1:1 co-culture medium of HeLa cells and 293FT cells was transfected with 90 ng of miR-21-5p-responsive hmAG mRNA and 180 ng of control tagBFP mRNA. As the RNAs, RNAs having m5C/Ψ modified base or m1Ψ modified base were used. Standardized translation efficiency (hmAG/tagBFP) was divided into two groups: a group showing miR-21-5p activity in HeLa cells (high activity) and a group showing miR-21-5p activity in 293FT cells (low activity) (Fig. 11(a)). From the histogram showing the translation efficiency, it was confirmed that there was a larger separation between the two peaks when the miR switch having the m1Ψ modified base was used (Fig. 11 (b)). It shows that multiple types of miRNA-responsive Off switch mRNAs composed of nucleotides having a modified base have improved the dynamic range in HeLa cells. 90 ng of miR-21-5p-responsive hmAG mRNA (miR-21-5p target sequences 1x and 4x) and 180 ng of m5C/Ψ modified control tagBFP mRNA were transfected into HeLa cells with Lipofectamine MessengerMAX. From this result, it was found that an mRNA switch consisting of a nucleotide sequence having a m1Ψ modified base greatly improves the dynamic range of switch-on/off defined above.

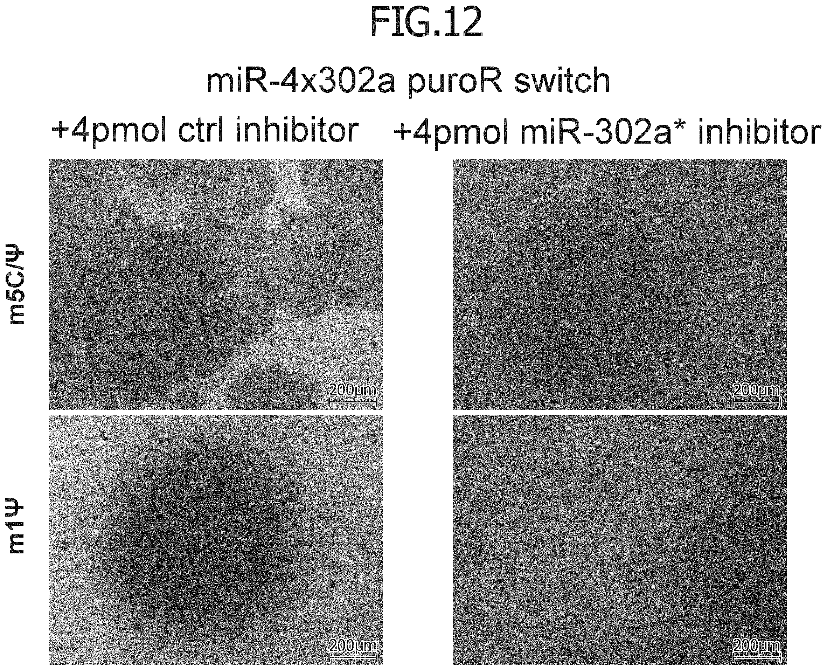

Fig. 12 is photographs of an iPSC stained with alkaline phosphatase (ALP). 201B7 cells were placed in a 24-well plate at 0.7 x 10⁵ cells/well. After 24 hours, 30 ng of miR-4x302a-responsive puroR mRNA composed of nucleotides having m5C/Ψ modified bases or m1Ψ modified base, and 4 pmol of a control inhibitor or miR-302a-5p inhibitor were transfected into cells. The puroR means a puromycin resistance gene. The miR-4x302a-responsive puroR mRNA is an mRNA switch having 4 repeats miR-302a-5p target sequence in the 5' UTR, and encoding puromycin. This switch suppresses puroR expression in response to miR-302a-5p expression. As the control inhibitor (ctrl inhibitor), mirVana(TM) miRNA Inhibitor, Negative Control #1 was used. After 6 hours, the medium was replaced with a medium supplemented with 2 µg/ml puromycin. The following day, the medium was replaced with normal iPSC medium and the cells were cultured for an additional 6 days to fix, and ALP staining was performed. As a result, suppression of puroR expression was confirmed in response to miR-302a-5p expression in 201B7 cells. Subsequently, puromycin treatment resulted in cell death of 201B7 cells that did not express puroR. Fig. 12 shows photographs of viable cells of 201B7 after 6 days of the ALP staining. This result revealed that the number of viable cells of 201B7 cells transfected with responsive mRNAs having a m1Ψ modified base was clearly small (lower left). Since miR-302a-5p inhibitors inhibit the activity of endogenous miR-302a-5p, cell death does not occur. Thus, it was confirmed that cell death was dependent on 302a-5p expression.

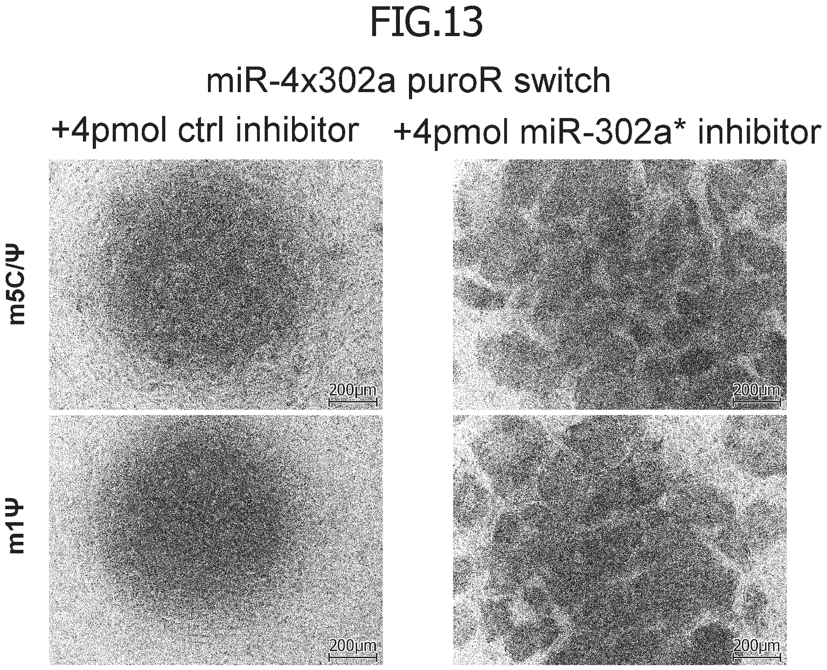

Fig. 13 is photographs of partially differentiated iPSCs stained with alkaline phosphatase (ALP). iPSCs (Day 7) which partially differentiated from 201B7 cells were placed to iPSC medium in a 24-well plate at 0.7 x 10⁵ cells/well. After 24 hours, 30 ng of miR-4x302a-puroR mRNA composed of nucleotides having m5C/Ψ modified bases or m1Ψ modified base, and 4 pmol of control inhibitor or miR-302a-5p inhibitor were transfected into cells. After 6 hours, the medium was exchanged with a medium supplemented with 2 µg/ml puromycin. The following day, the medium was exchanged with normal iPSC medium and the cells were cultured for an additional 6 days to fix, and ALP staining was performed. From the result of the ALP staining, it was found that the number of viable cells of 201B7 cells was clearly small in cells transfected with responsive mRNAs having a m1Ψ modified base. It was found that responsive mRNAs having a m1Ψ modified base greatly improves the ability to specifically remove cells depending on endogenous miRs.

Furthermore, mRNA switches composed of m1Ψ modified bases, prepared using the templates shown in Table 6 of Examples, which specifically responding to miR-661, miR-335, and miR-210, also showed high expression in viable cells compared with mRNA switches having normal uridine, and improved dynamic range.

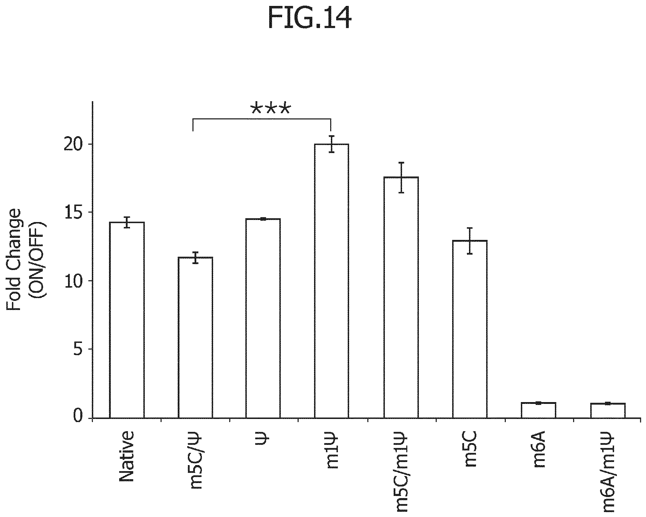

The miR-21-5p-responsive EGFP switches each consisting of a nucleotide sequence having native or a seven-type varied modified base were prepared and transfected into 293FT cells in the same manner as described in the "Transfection". Fig. 14 is a graph showing the results of expressing miR-21-5p-responsive EGFP switches each consisting of a nucleotide sequences having a varied modified base in 293FT cells. The switch-off state was achieved by co-introducing a miR-21-5p mimic with the miR-21-5p-responsive EGFP switch. Fold Change was calculated by dividing the translation efficiency of the miR-21-5p-responsive EGFP switch in the switch-on state by the translation efficiency of that in the switch-off state. The Fold Change is used in the same meaning as the dynamic range described above. In the graph, Native represents an mRNA consisting of a native nucleotide sequence having no modified bases, and Ψ, m1Ψ, m5C/Ψ, m5C/m1Ψ, m5C, m6A, and m6A/m1Ψ are mRNAs each consisting of a nucleotide sequence having a modified base(s) described in the *"In vitro* Transcription". The measurement was performed three times, and the data was expressed as mean +/- standard error.

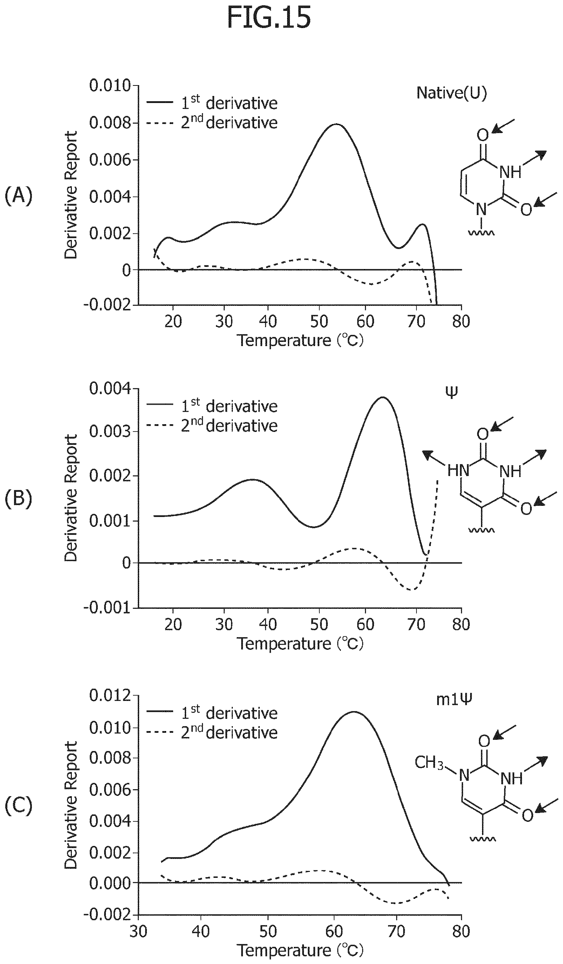

The melting temperature Tₘ of a native oligonucleotide and oligonucleotides having Ψ or m1Ψ modified bases was examined. Tₘ oligonucleotides were synthesized by IVT using a primer set of T7_fwd TAATACGACTCACTATAG and T7_rev CGCAAAACGCCTATAGTGAGTCGTATTA shown in Table 9 below. The resulting RNA product was purified by PAGE and gel extraction. Reverse complementary nucleotides were purchased from GeneDesign Inc. (Osaka, Japan). A UV melting experiment was performed using a spectrophotometer (JASCO V-650 UV/VIS) equipped with a temperature controller and 8 microcell holders (JASCO PAC-743R). First, equimolar concentrations of each oligonucleotide (final concentration 4 µM) and RNA complementary strands were gradually cooled from 85°C to room temperature in a pH 7.0 buffer containing 20 mM sodium phosphate and 50 mM NaCl using a ProFlex(TM) PCR system (Thermo Fisher Scientific). The melting profile was scanned at 0.5°C/min in the temperature range of 15°C to 85°C at a wavelength of 260 nm. Tₘ was calculated at the temperature at which the duplex is half-dissociated, and each Tₘ was determined from derivatives of the melting curve. Table 10 below indicates Tₘ values of native oligonucleotide, m1Ψ oligonucleotide, and Ψ oligonucleotide, each annealed with the native complementary oligo CGCAAAACGCC. The measurement was performed three times, and the data was expressed as mean +/standard error. Tₘ of possible unstable forms were also measured. Figs. 15(A), 15(B) and 15(C) show typical first and second derivative reports of the native oligonucleotide, Ψ oligonucleotide, and m1Ψ oligonucleotide, respectively. The arrows indicate possible hydrogen binding sites on the nucleobases. It is believed that, since Ψ has many potential hydrogen bonding sites, it easily forms unstable forms.

**[Table 9]**

| **Primer** | **Sequence** 5'**to** 3' | SEQ ID NO: |
|---|---|---|
| YK_T7_fwd short | TAATACGACTCACTATAG | 55 |
| YK_T7_rev | cgcaaaacgcCTATAGTGAGTCGTATTA | 56 |

**[Table 10]**

| **Type** | **Sequence** | Tₘ(°C) |
|---|---|---|
| Native | GGCGUUUUGCG | 54.8 ± 1.2 |
| m1Ψ | GGCGm1Ψm1Ψm1Ψm1ΨGCG | 63.5 ± 0.1 |
| Ψ | GGCΨΨΨΨGCG | 63.3 ± 0.5 *38.5± 4,8 |

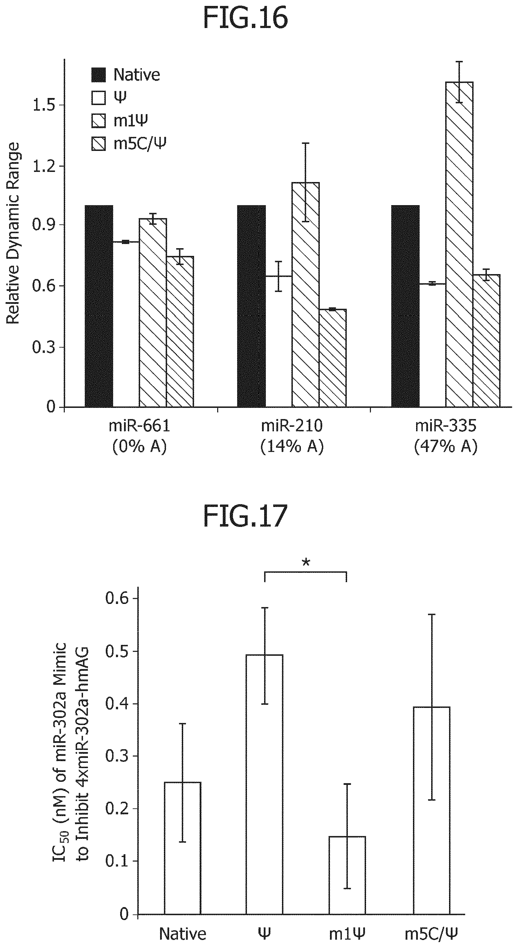

The relationship between the amount of m1Ψ in the target sequence of miRNA-responsive mRNA switch and the translation efficiency was examined. The miR-661-responsive EGFP switch, miR-210-responsive EGFP switch, and miR-3 3 5-responsive EGFP switch, each consisting of a nucleotide sequence having native, or a Ψ, m1Ψ, or m5C/Ψ modified base(s), were prepared and transfected to 293FT cells. Table 11 below shows each sequence of miR-661, miR-210 and miR-335, and complementary sequences of miR-661, miR-210, and miR-335. In the table, U in the complementary sequence is indicated in bold. Seed sequences and their complementary sequences are underlined. 3' compensatory sites and their complementary sequences are indicated by a dotted underline. Fig. 16 is a graph showing a relative dynamic range of the miR-661-responsive EGFP switch, miR-210-responsive EGFP switch, or miR-3 3 5-responsive EGFP switch each containing native or a Ψ, m1Ψ, or m5C/Ψ modified base(s) when the dynamic range of the switch consisting of native oligonucleotide is set to 1. In the graph, Native represents an mRNA consisting of a native nucleotide sequence having no modified bases, and Ψ, m1Ψ, and m5C/Ψ are mRNAs each consisting of a nucleotide sequence having a modified base(s) described in the *"In vitro* Transcription". The measurement was performed three times, and the data was expressed as mean +/- standard error. From this data, it was found that the effect of m1Ψ that promotes miRNA switches compared to Ψ and native U depends on the amount of U contained in the miRNA target sequence.

**[Table 11]**

| **microRNA** | **microRNA ID (miRBase v21)** | **Sequence** | **Complementary Sequence** |
|---|---|---|---|
| hsa-miR-661 | MIMAT0003324 | ugccugggucucuggccugcgcgu | acgcgcaggccagagacccaggca |
| hsa-miR-210-3p | MIMAT0000267 | cugugcgugugacagcggcuga | ucagccgcugucacacgcacag |
| hsa-miR-335-5p | MIMAT0000765 | ucaagagcaauaacgaaaaaugu | acauuuuucguuauugcucuuga |

4xmiR-302a-hmAG mRNAs, each consisting of a nucleotide sequence having native or a Ψ, m1Ψ or m5C/Ψ modified base(s), were prepared. Along with 90 ng of 4xmiR-302a-hmAG mRNA and 90 ng of control mRNA, 3 to 0.0003 nM miRNA mimic was co-introduced into 293FT cells. The cells were measured by flow cytometry with AccuriC6 or Aria (BD Biosciences) 24 hours after the introduction. The mean fluorescence intensity of the marker protein encoded by 4xmiR-302a-hmAG mRNA in each cell was normalized by the mean intensity of the control mRNA. The percentage of suppression by the miRNA mimic was calculated by comparing the mean fluorescence intensity of the normalized marker protein in each sample with the mean fluorescence intensity of the normalized marker protein in the sample not containing the miRNA mimic. Fig. 17 is a graph showing the amount of miR-302a mimics required to suppress 50% of the expression in 293FT cells of 4xmiR-302a-hmAG mRNA switches having native, or a Ψ, m1Ψ, or m5C/Ψ modified base(s). In the graph, Native represents an mRNA consisting of a native nucleotide sequence having no modified bases, and Ψ, m1Ψ, and m5C/Ψ are mRNAs each consisting of a nucleotide sequence having a modified base(s) described in the *"In vitro* Transcription". The measurement was performed three times, and the data was expressed as mean +/- standard error. This data showed that a switch consisting of a nucleotide sequence having a m1Ψ modified base has a higher binding affinity with the miR mimic than the other switches.

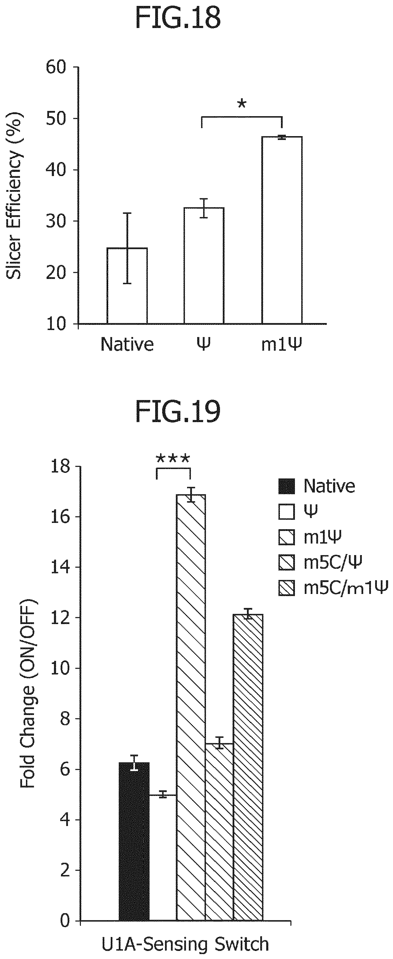

293FT cells were placed the day before transfection in a 12-well plate at a concentration of 1 x 10⁵ cells/well. The miR-335-EGFP switch mRNA (200 ng) of Native, Ψ or m1Ψ and control Renilla RNA (30 ng), control or miR-335 mimic (3 nM) were introduced into 293FT cells with Lipofectamine MessengerMax. 4 hours after the introduction, the medium was replaced with fresh medium. 8 hours after the introduction, cells were washed with 1 mL of RT PBS buffer and then added to TRIzol(TM). Duplicate sets of cells were trypsinized and measured with Accuri. Total RNA of cells was generated by miRNeasy protocol. Using 100 ng of total RNA, cDNA was produced (ReverTra Ace(R); 0.2 ng of firefly mRNA was added to each sample as RT control). The amount of the remaining switches was measured by standard SYBR Green RT-qPCR. Fig. 18 shows that the miR-loaded RISC decomposes the m1Ψ switch more efficiently than the native and Ψ switches. 8 hours after the transfection, Slicer efficiency of various miR-335-EGFP mRNA switches in 293FT cells was measured. The Slicer efficiency is an indicator expressed as a percentage of cleaved switches in cells induced by co-introduction of a miR mimic and miRNA switch. In the graph, Native represents an mRNA consisting of a native nucleotide sequence having no modified bases, and m1Ψ and Ψ are mRNAs each consisting of a nucleotide sequence having a modified base described in the *"In vitro* Transcription". The measurement was performed three times, and the data was expressed as mean +/- standard error.

U1A-responsive EGFP mRNA switches containing different modified bases were prepared. 200 ng of the U1A-responsive EGFP mRNA switch and 90 ng of the control iRFP670 mRNA were co-introduced into 293FT cells with 100 ng of U1An mRNA or without U1An mRNA. The cells were measured by flow cytometry with Accuri C6 (BD Biosciences) 8 or 24 hours after the transfection. Fig. 19 shows the Fold change in 293FT cells of the U1A-responsive EGFP mRNA switches containing different modified bases. The translation efficiency when U1An mRNA is introduced was defined as a switch-off state, and the translation efficiency when U1An mRNA was not introduced was defined as a switch-on state, then the Fold Change was calculated by dividing the translation efficiency in the switch-on state by the translation efficiency in the switch-off state. In the graph, Native represents an mRNA consisting of a native nucleotide sequence having no modified bases, and Ψ, m1Ψ, m5C/Ψ, and m5C/m1Ψ are mRNAs each consisting of a nucleotide sequence having a modified base(s) described in the *"In vitro* Transcription". The measurement was performed three times, and the data is expressed as mean +/- standard error.

## Claims

1. An mRNA comprising: (i) a nucleic acid sequence specifically recognized by a miRNA or protein; and (ii) a nucleic acid sequence corresponding to a coding region for a marker protein, wherein a nucleotide contained in the mRNA comprises N¹-methyl pseudouridine.

2. The mRNA according to claim 1, wherein the nucleic acid sequence of (i) is a nucleic acid sequence specifically recognized by a miRNA, and the nucleic acid sequences of (i) and (ii) are linked to each other in a 5' to 3' direction.

3. The mRNA according to claim 1, wherein the nucleic acid sequence of (ii) is linked to the 3' side of the nucleic acid sequence of (i), and the mRNA further comprises a translation suppression sequence at the 3' side of the nucleic acid sequence of (ii).

4. The mRNA according to claim 1, wherein the nucleic acid sequence of (i) is a nucleic acid sequence specifically recognized by a protein, and the nucleic acid sequences of (i) and (ii) are linked to each other in a 5' to 3' direction.

5. The mRNA according to any one of claims 1 to 4, wherein the marker protein is a membrane protein or a secretory protein.

6. The mRNA of any one of claims 1 to 4, wherein the marker protein is a fluorescent protein, a luminescent protein, a drug-resistant protein, a cell death-inducible protein, or a cell death-inhibitory protein.

7. A method for distinguishing a cell, comprising the steps of:
(1) introducing an mRNA into the cell, wherein the mRNA comprises (i) a nucleic acid sequence specifically recognized by a miRNA or protein and (ii) a nucleic acid sequence corresponding to a coding region for a marker protein, and wherein a nucleotide contained in the mRNA comprises N¹-methyl pseudouridine; and
(2) distinguishing a cell type using a translation amount of the marker protein as an indicator.

8. The method according to claim 7, wherein the marker protein is a membrane protein or a secretory protein.
